(19) Europäisches Patentamt
European Patent Office
Office européen des brevets

(11) Veröffentlichungsnummer: **0 215 307 B1**

(12) # EUROPÄISCHE PATENTSCHRIFT

(45) Veröffentlichungstag der Patentschrift: **24.07.91**

(51) Int. Cl.$^5$: **C07D 405/04**, A01N 43/54

(21) Anmeldenummer: **86111367.8**

(22) Anmeldetag: **18.08.86**

(54) **Pyrimiden-Derivate, Verfahren zu ihrer Herstellung und ihre Verwendung als Mikrobizide.**

(30) Priorität: **21.08.85 CH 3587/85**
**25.04.86 CH 1694/86**

(43) Veröffentlichungstag der Anmeldung:
**25.03.87 Patentblatt 87/13**

(45) Bekanntmachung des Hinweises auf die Patenterteilung:
**24.07.91 Patentblatt 91/30**

(84) Benannte Vertragsstaaten:
**AT BE CH DE FR GB IT LI LU NL SE**

(56) Entgegenhaltungen:

**CHEMICAL ABSTRACTS, Band 79, 1973, Seite 330, Zusammenfassung Nr. 146551h, Columbus, Ohio, US; & JP-A-73 49 776 (SHIONOGI AND CO., LTD) 13-07-1973**

(73) Patentinhaber: **CIBA-GEIGY AG**
**Klybeckstrasse 141**
**CH-4002 Basel(CH)**

(72) Erfinder: **Eckhardt, Wolfgang, Dr.**
**Breslauerstrasse 14**
**W-7850 Lörrach(DE)**
Erfinder: **Huxley, Philip, Dr.**
**Bleichestrasse 7/2**
**CH-4058 Basel(CH)**

(74) Vertreter: **Zumstein, Fritz, Dr. et al**
**Bräuhausstrasse 4**
**W-8000 München 2(DE)**

**EP 0 215 307 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft substituierte Aryl-dioxolan-pyrimidine und Aryl-dioxan-pyrimidine der nachstehenden Formel I sowie deren Säureadditionssalze. Sie betrifft ferner die Herstellung dieser Verbindungen sowie agrochemische Mittel, die als Wirkstoff mindestens eine der Verbindungen der Formel I enthalten, die Herstellung dieser Mittel und ein Verfahren zur Bekämpfung oder Verhütung des Befalls von Pflanzen durch phytopathogene Mikroorganismen.

Die erfindungsgemässen Verbindungen entsprechen der allgemeinen Formel I

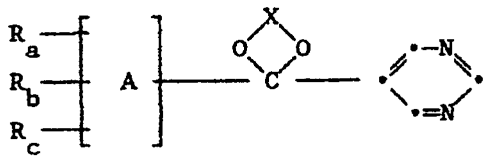

(I) ,

in welcher

A für Phenyl, Phenoxyphenyl, Phenylthiophenyl, Biphenyl oder Naphthyl steht,

$R_a$, $R_b$ und $R_c$ unabhängig voneinander Wasserstoff, Halogen, Cyano, Nitro, $C_1$-$C_4$-Alkyl, $C_1$-$C_4$-Alkoxy, $C_1$-$C_4$-Haloalkyl oder $C_1$-$C_4$-Haloalkoxy darstellt, und

X eines der folgenden Brückenelemente

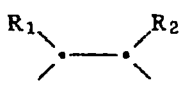

oder

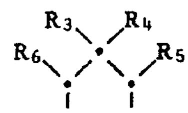

bedeutet, worin $R_1$ und $R_2$ unabhängig voneinander Wasserstoff, $C_1$-$C_4$-Alkyl, $C_1$-$C_4$-Haloalkyl oder $CH_2O$-$C_1$-$C_4$-Alkyl darstellen und

$R_4$ Wasserstoff, $C_1$-$C_4$-Alkyl oder $CH_2OH$ und

$R_3$, $R_5$, und $R_6$ unabhängig voneinander Wasserstoff oder $C_1$-$C_4$-Alkyl bedeuten, und schliessen ihre Säureadditionssalze mit ein.

Im engeren Sinne betrifft die Erfindung Verbindungen der Formel I, worin A, $R_a$, $R_b$, $R_c$, $R_1$ und $R_2$ die vorgenannte Bedeutung haben und worin $R_6$ Wasserstoff bedeutet, während $R_3$, $R_4$ und $R_5$ unabhängig voneinander Wasserstoff oder $C_1$-$C_4$-Alkyl bedeuten, unter Einschluss ihrer Säureadditionssalze (Wirkstoffgruppe Ia).

Unter Alkyl selbst oder als Bestandteil eines anderen Substituenten sind je nach Zahl der angegebenen Kohlenstoffatome beispielsweise folgende Gruppen zu verstehen; Methyl, Ethyl, Propyl, Butyl sowie die Isomeren Isopropyl, Isobutyl, tert.-Butyl oder sek.-Butyl.

Halogen selbst oder als Bestandteil eines anderen Substituenten bedeutet Fluor, Chlor, Brom oder Jod, vorzugsweise Fluor, Chlor oder Brom.

Die Brückenelemente X bilden mit den Sauerstoffatomen und dem gemeinsamen C-Atom Dioxolan- und Dioxan-Ringe.

Die Erfindung betrifft sowohl die freien Verbindungen der Formel I, als auch deren Additionssalze mit organischen und anorganischen Säuren.

Beispiele salzbildender Säuren sind anorganische Säuren: Halogenwasserstoffsäuren wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Weinsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Die Verbindungen der Formel I sind bei Raumtemperatur stabile Oele, Harze oder Feststoffe, die sich durch sehr wertvolle mikrobizide, insbesondere pflanzenfungizide Eigenschaften auszeichnen. Sie lassen sich daher bevorzugt auf dem Agrarsektor oder verwandten Gebieten präventiv und kurativ zur Bekämpfung von phytopathogenen Mikroorganismen einsetzen.

Aufgrund ihrer ausgeprägten mikrobiziden Wirkung sind diejenigen Wirksubstanzen der Formel I bevorzugt, die folgende Substituenten oder Kombinationen dieser Substituenten untereinander aufweisen:

für A :
a) Phenyl, Phenoxyphenyl oder Phenylthiophenyl; besonders
b) Phenyl oder Phenoxyphenyl;
für $R_a$, $R_b$ und $R_c$ unabhängig voneinander:
a) Wasserstoff, Halogen, $C_1$-$C_2$-Alkyl, $C_1$-$C_2$-Alkoxy, $C_1$-$C_2$-Haloalkyl, $C_1$-$C_2$-Haloalkoxy, Nitro oder Cyano; besonders
b) Wasserstoff, Halogen, $C_1$-$C_2$-Alkyl, $C_1$-$C_2$-Alkoxy, $C_1$-$C_2$-Haloalkoxy, Nitro oder Cyano;
CCCCC38
für X:

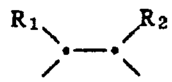 oder 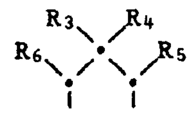 ;

für $R_1$ und $R_2$ unabhängig voneinander:
a) Wasserstoff, $C_1$-$C_4$-Alkyl, $C_1$-$C_2$-Haloalkyl oder $CH_2O$-$C_1$-$C_2$-Alkyl; besonders
b) Wasserstoff, $C_1$-$C_4$-Alkyl oder $CH_2OCH_3$;
für $R_4$:
a) Wasserstoff, $C_1$-$C_2$-Alkyl oder $CH_2OH$; besonders
b) Wasserstoff, Methyl oder $CH_2OH$;
für $R_3$, $R_5$ und $R_6$ unabhängig voneinander:
a) Wasserstoff oder $C_1$-$C_2$-Alkyl; besonders
b) Wasserstoff und/oder Methyl.

Für die Wirkstoffgruppe Ia gelten die gleichen Bevorzugungen für A, $R_a$, $R_b$ und $R_c$, während X bevorzugt

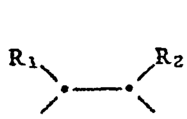 oder 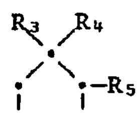

bedeutet; $R_1$ und $R_2$ unabhängig voneinander
a) Wasserstoff, $C_1$-$C_4$-Alkyl, $C_1$-$C_2$-Haloalkyl oder $CH_2O$-$C_1$-$C_2$-Alkylsind; besonders bevorzugt
b) Wasserstoff, $C_1$-$C_4$-Alkyl oder $CH_2OCH_3$; und
$R_3$, $R_4$ und $R_5$ unabhängig voneinander Wasserstoff oder $C_1$-$C_2$-Alkyl darstellen.

Aus dem Umfang der oben aufgeführten Substituentenkombinationen sind solche Verbindungen der Formel I bzw. der Wirkstoffgruppe Ia besonders bevorzugt, die über folgende Substituenten oder Substituentenkombinationenverfügen:
für A: Phenyl oder Phenoxyphenyl;
für $R_a$, $R_b$ und $R_c$ : unabhängig voneinander Chlor oder Methyl, insbesondere in 2- und/oder 4-Stellung;
für X:

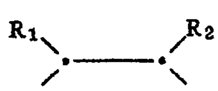

für $R_1$: $C_1$-$C_3$-Alkyl oder $CH_2OCH_3$;
für $R_2$: Wasserstoff;
Es sind ferner Verbindungen der Formel I bevorzugt, bei denen A in Kombination mit $R_a$, $R_b$ und $R_c$ 2,4-Dichlorphenyl, 2-Fluor-4-chlorphenyl, 2-Cyano-4-chlorphenyl, 2-Methyl-4-chlorphenyl, p-Chlorphenoxy, 2-Methyl-4-(p-chlorphenoxy) oder 2-Chlor-4-(p-chlorphenoxy) bedeutet, während $R_1$ bis $R_6$ Wasserstoff oder $C_1$-$C_4$-Alkyl darstellen, zusammen aber höchstens 5 C-Atome besitzen.

Die Verbindungen der Formel I können wie folgt hergestellt werden:

Methode A:

In einer Ketalisierungsreaktion wird ein Keton der Formel II

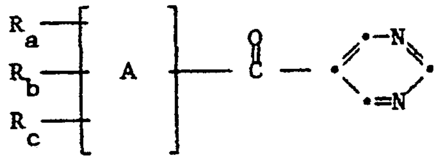 (II)

mit einem Diol der Formel III

HO - X - OH (III) ,

in welchen A, $R_a$, $R_b$, $R_c$ und X die unter Formel I angegebenen Bedeutungen besitzen, in einem inerten Lösungsmittel gegebenenfalls in Gegenwart einer Säure umgesetzt.

Methode B:

Ein Keton der Formel IV

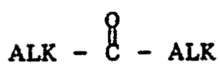 (IV)

wird mit einem Diol der Formel III in einem inerten Lösungsmittel gegebenenfalls in Gegenwart einer Säure zu einer Verbindung der Formel V

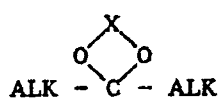 (V)

worin ALK ein $C_1$-$C_4$-Alkyl darstellt und X die unter Formel I angegebenen Bedeutungen besitzt, umgesetzt, wobei direkt anschliessend ohne Isolierung der gebildeten Verbindung der Formel V mit dieser die Umketalisierung eines Ketons der Formel II in einem inerten Lösungsmittel gegebenenfalls in Gegenwart einer Säure durchgeführt wird.

Diese Reaktionen können analog zu bekannten Ketalisierungsreaktionen durchgeführt werden, beispielsweise analog zur Herstellung von 2-Brommethyl-2,4-diphenyl-1,3-dioxolan [Synthesis, 1974 (I), 23].

In einer bevorzugten Ausführung der Ketalisierung werden beide Reaktionspartner mehrere Stunden zusammen mit einem Azeotrop-Bildner in einem der üblichen organischen Lösungsmittel unter Rückfluss erhitzt. Als Azeotrop-Bildner kommen z.B. Benzol, Toluol, Xylol, Chloroform oder Tetrachlorkohlenstoff in Frage, wobei zur Beschleunigung der Reaktion ein Zusatz einer starken Säure, wie z.B. p-Toluolsulfonsäure vorteilhaft sein kann. Verwendbare organische Lösungsmittel sind in diesem Fall z.B. aromatische Kohlenwasserstoffe, wie Benzol, Toluol, Xylol usw. und gesättigte Kohlenwasserstoffe, wie n-Hexan. Als Reaktionsbeschleuniger können ferner anorganische oder organische Säuren verwendet werden. Beispiele anorganischer Säuren sind Chlorwasserstoffsäure, Bromwasserstoffsäure, Jodwasserstoffsäure, Schwefelsäure, Phosphorsäure, phosphorige Säure, Salpetersäure. Beispiele organischer Säuren sind Essigsäure, Trichloressigsäure, Oxalsäure, Benzolsulfonsäure, Methansulfonsäure, p-Toluolsulfonsäure.

Die Reaktionstemperaturen der Ketalisierungsreaktionen betragen 20° bis 180° C, vorzugsweise 40° bis 150° C.

Die beschriebenen Verfahren stellen einen Bestandteil der vorliegenden Erfindung dar.

Bei den vorstehend beschriebenen Ketalisierungsreaktionen von Ketonen mit substituierten $\alpha,\beta$- oder $\alpha,\gamma$-Diolen entstehen Gemische von Diastereomeren der resultierenden Ketale, sofern mindestens einer der Substituenten $R_1$ bis $R_6$ eine andere Bedeutung als Wasserstoff hat. Entsprechend bilden sich aus den Ausgangsketonen im allgemeinen Diastereomerengemische der Endprodukte der Formel I.

Die Trennung der beiden Diastereomeren und Enantiomeren kann auf übliche Weise erfolgen. Die

beiden Isomeren können unterschiedliche mikrobizide Wirkung aufweisen. Im allgemeinen werden für praktische Zwecke die Diastereomerengemische verwendet.

Die Erfindung betrifft sämtliche isomeren Verbindungen der Formel I und deren Salze.

Wegen ihrer biologischen Aktiviät werden folgende Einzelverbindungen sowohl als einzelnes Isomeres A oder B als auch als Isomeren-Gemisch (A,B) bevorzugt.

5-[2-(2',4'-Dichlorphenyl)-4-ethyl-1,3-dioxolan-2yl]-pyrimidin [Verb. 1.3a,b; 1.3a; 1.3b]
5-[2-(2',4'-Dichlorphenyl)-4-propyl-1,3-dioxolan-2-yl]-pyrimidin [Verb. 1.4a; 1.4b; 1.4a,b]
5-[2-(2',4'-Dichlorphenyl)-4-methoxymethyl-1,3-dioxolan-2yl]-pyrimidin [Verb. 1.7a; 1.7b; 1.7a,b]
5-[2-(2',4'-Dichlorphenyl)-5-ethyl-5-methyl-1,3-dioxan-2yl]-pyrimidin [Verb. 2.19a,b; besonders 2.19a]
5-[2-(2',4'-Dichlorphenyl)-4-methyl-1,3-dioxan-2yl]-pyrimidin [Verb. 3.6a,b]
5-[2-(2',4'-Dichlorphenyl)-4,5-dimethyl-1,3-dioxolan-2yl]-pyrimidin[Verb. 1.6a; 1.6b; 1.6a,b]
5-[2-(2',4'-Dichlorphenyl)-5,5-dimethyl-1,3-dioxan-2yl]-pyrimidin [Verb. 2.2]
5-[2-(2',4'-Dichlorphenyl)-4,6-dimethyl-1,3-dioxan-2yl]-pyrimidin [Verb. 3.1]
5-[2-(2',4'-Dichlorphenyl)-4,5,6-trimethyl-1,3-dioxan-2yl]-pyrimidin [Verb. 3.2]
5-[2-(2'-Cyano-4'-chlorphenyl)-5-ethyl-5-methyl-1,3-dioxan-2yl]-pyrimidin [Verb. 2.23a,b]
5-[2-(2',4'-Dichlorphenyl)-4-ethyl-1,3-dioxan-2yl]-pyrimidin [Verb. 3.11a,b]
5-[ 2-(2'-Chlor-4'-p-chlorphenoxiphenyl)-4-ethyl-1,3-dioxan-2yl]-pyrimidin [Verb. 2.25a,b].

Die zur Herstellung der erfindungsgemässen Endprodukte der Formel I verwendeten Ausgangsstoffe der Formel II sind teilweise neu und stellen, soweit sie neu sind, einen Bestandteil der vorliegenden Erfindung dar. Sie können nach an sich bekannten Verfahren hergestellt werden, wie z.B. durch Umsetzung von Carbonsäureestern der Formel

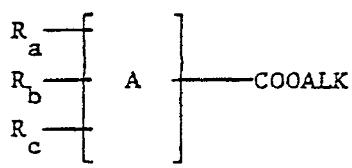

mit Halo-pyrimidinen der Formel

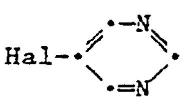

in welchen ALK $C_1$-$C_4$-Alkyl, vorzugsweise Methyl oder Ethyl, und Hal Halogen, vorzugsweise Chlor oder Brom, darstellen und A, $R_a$, $R_b$ und $R_c$ die unter Formel I angegebenen Bedeutungen besitzen, in inerten organischen Lösungsmitteln in Gegenwart von n-Butyllithium bei Temperaturen von -150° bis 25° C.

Als Aryl-dioxolan-pyrimidin-Derivat ist beispielsweise in der Literatur (s. Jap. Kokai 73 49,776; Chemical Abstracts, Band 79, 1973, Seite 330, Nr.146551 h) folgende Verbindung beschrieben

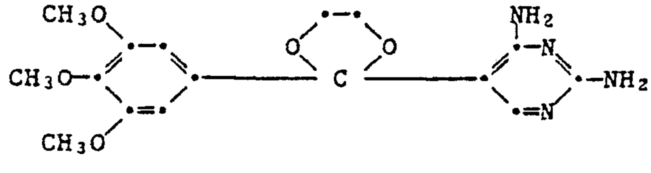

Dieser Strukturtyp ist als Potentiator für Sulfonamide zur Bekämpfung von Infektionskrankheiten bei Säugetieren bekannt.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende

Pflanzenteile von derartigen Mikroorganismen verschont bleiben.

Die Wirkstoffe der Formel I sind gegen die den folgenden Klassen angehörenden phytopathogenen Pilze wirksam: Fungi imperfecti (z.B. Botrytis, Helminthosporium, Fusarium Septoria, Cercospora, Pyricularia, Alternaria); Basidiomyceten (z.B. die Gattungen Hemileia, Rhizocotonia, Puccinia); insbesondere wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüber hinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Grapefruit, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (=Samenbeizung). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise

hergestellt, z.B. durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon,Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z.B. Phosphatidylchlorin-Mischungen. Synthetische Phospholipide sind z.B. Dioctanoylphosphatidylchlolin und Dipalmitoylphophatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. Das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat

in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten. Derartige agrochemische Mittel sind ein Bestandteil der vorliegenden Erfindung.

Die nachfolgenden Beispiele dienen zur näheren Erläuterung der Erfindung, ohne dieselbe einzuschränken.

## 1. Herstellungsbeispiele

Beispiel 1.1: Herstellung vom

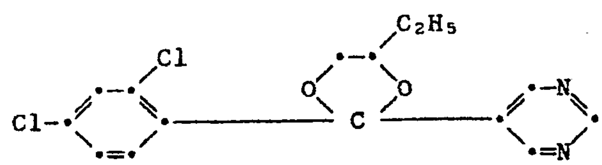

5-[2-(2',4'-Dichlorphenyl)-4-ethyl-1,3-dioxolan-2-yl]-pyrimidin

41,6 g (0,4 Mol) Acetondimethylacetal und 36 g (0,4 Mol) 1,2-Butandiol und eine Spatelspitze p-Toluolsulfonsäure werden 2 Stunden in 300 ml 1,1,1-Trichloräthan unter Rückfluss am Wasserabscheider gerührt. Nach dem Abkühlen werden 10,1 g (0,04 Mol) 5-(2,4-Dichlorphenyl)-ketopyrimidin und 15,2 g (0,08 Mol) p-Toluolsulfonsäure zugegeben und das Reaktionsgemisch 24 Std. am Wasserabscheider bei Siedetemperatur gerührt. Danach werden weitere 7,2 g (0,08 Mol) 1,2-Butandiol und 4 Tropfen konz. Schwefelsäure zugegeben und über Nacht am Rückfluss gerührt. Anschliessend werden nochmals 7,2 g (0,08 Mol) 1,2-Butandiol und 4 Tropfen konz. Schwefelsäure zugegeben und weitere 16 Stunden bei Siedetemperatur am Wasserabscheider gerührt. Nach dem Abkühlen wird das Reaktionsgemisch nacheinander 3 mal mit 1 N Natronlauge und 1 mal mit Wasser extrahiert, die organische Phase über Natriumsulfat getrocknet, filtriert und eingeengt. Das Rohprodukt wird über eine Kieselgel-Flash-Säule mit Petroläther/Essigsäureethylester (3:1) gereinigt. Man erhält so 0,6 g eines Isomeren A ($n_D^{40}$ 1,5528) und 0,35 g eines Isomeren B ($n_D^{40}$ 1,5634) sowie 1,5 g Isomerengemisch ($n_D^{40}$ 1,5654).

Beispiel 1.2: Herstellung von

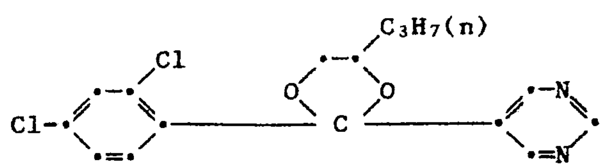

5-[2',4'-Dichlorphenyl)-4-n-propyl-1,3-dioxolan-2-yl]-pyrimidin

In einem Dreihalskolben mit aufgesetztem Soxhlet, der mit 100 g Molekularsieb (0,5 mm) gefüllt ist, werden 8,9 g (0,035 Mol) 5-(2,4-Dichlorbenzoylpyrimidin, 5,5 g (0,0525 Mol) 1,2-Pentandiol, 14 g (0,0735

Mol) p-Toluolsulfonsäure und 35 ml n-Butanol in 300 ml Toluol während 20 Stunden bei Siedetemperatur (Badtemperatur 160°C) gerührt. Nach dem Abkühlen auf Raumtemperatur wird das Reaktionsgemisch 3-mal mit Wasser extrahiert, anschliessend über Natriumsulfat getrocknet, filtriert und am Wasserstrahlvakuum eingeengt. Das Rohprodukt wird über eine Flash-Kieselgelsäule mit Essigsäureethylester/Petrolether (1:3) gereinigt. Man isoliert so 3,1 g eines Isomeren A ($n_D^{40}$ 1,5522), 3,4 g eines Isomeren B ($n_D^{40}$ 1,5578) und 0,6 g Isomerengemisch A/B ($n_D^{40}$ 1,5551). Die Gesamtausbeute beträgt 7,1 g (60 % d.Th.).

Beispiel 1.3: Herstellung von

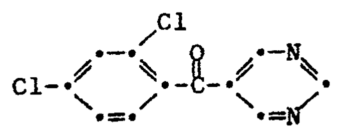

5-(2',4'-Dichlorbenzoyl)-pyrimidin

61,8 g (0,3 Mol) 2,4-Dichlorbenzoesäuremethylester und 52,2 g (0,33 Mol) 5-Brompyrimidin werden in einem Gemisch von 400 ml Tetrahydrofuran und 200 ml Diethylether gelöst, mit 30 g Molekularsieb versetzt und 0,5 Stdn bei Raumtemperatur gerührt. Dann wird das Reaktionsgemisch auf ca. -120°C abgekühlt und 187,2 ml (0,3 Mol) Buthyllithium als 1,6 molare Lösung in Hexan bei dieser Temperatur zugetropft. Das Reaktionsgemisch wird dann 1,5 Stdn. bei -100°C bis -130°C gerührt und anschliessend bei bis auf Raumtemperatur steigende Temperatur zu Ende gerührt. Nun werden 300 ml 10 %ige Ammoniumchloridlösung zugetropft und das Reaktionsgemisch mit Essigsäureethylester 3-mal extrahiert. Die organische Lösung wird über Natriumsulfat getrocknet, dann filtriert und eingeengt. Das erhaltene Rohprodukt wird dann über eine Flash-Kieselgelsäule mit Petrolether/Essigsäureethylester (3:1) als Fliessmittel gereinigt. Man erhält so 45 g (59,3 % d.Th.) der Titelverbindung vom Smp. 75-78°C.

Analog den vorstehend beschriebenen Beispielen 1.1 und 1.2 lassen sich die nachfolgend aufgeführten erfindungsgemässen Verbindungen - falls nicht besonders vermerkt - als Diastereomerengemische mit unterschiedlichen Mischungsverhältnissen herstellen. Die erhaltenen Isomeren A, B und C sind durch physikalische Konstanten charakterisiert. Sie lassen keinen Rückschluss auf die absolute Konfiguration zu. (In den Tabellen bedeutet:

Ph = 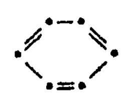 ;

Schmelzpunkte sind in Celsiusgraden angegeben.)

Tabelle 1: Verbindungen der Formel

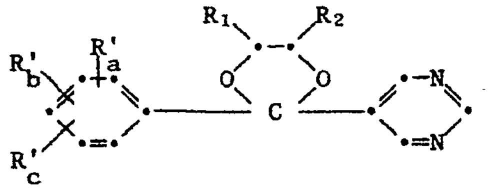

| Verb. Nr. | $R'_a$ | $R'_b$ | $R'_c$ | $R_1$ | $R_2$ | Physikal-Konstante |
|---|---|---|---|---|---|---|
| 1.1 | 2-Cl | 4-Cl | H | H | H | $n_D^{40}$ 1,5798 |
| 1.2 | 2-Cl | 4-Cl | H | $CH_3$ | H | $n_D^{40}$ 1,5722 |
| 1.3a | 2-Cl | 4-Cl | H | $C_2H_5$ | H | $n_D^{40}$ 1,5528 (Isomeres A) |
| 1.3b | 2-Cl | 4-Cl | H | $C_2H_5$ | H | $n_D^{40}$ 1,5634 (Isomeres B) |
| 1.3a,b | 2-Cl | 4-Cl | H | $C_2H_5$ | H | $n_D^{40}$ 1,5654 (Isomere A+B) |
| 1.4a | 2-Cl | 4-Cl | H | $C_3H_7(n)$ | H | $n_D^{40}$ 1,5522 (Isomeres A) |
| 1.4b | 2-Cl | 4-Cl | H | $C_3H_7(n)$ | H | $n_D^{40}$ 1,5578 (Isomeres B) |
| 1.4a,b | 2-Cl | 4-Cl | H | $C_3H_7(n)$ | H | $n_D^{40}$ 1,5557 (Isomere A+B) |
| 1.5 | 2-Cl | 4-Cl | H | $C_4H_9(n)$ | H | $n_D^{40}$ 1,5492 |
| 1.6a | 2-Cl | 4-Cl | H | $CH_3$ | $CH_3$ | Smp.92-96° (Isomeres A) |
| 1.6b | 2-Cl | 4-Cl | H | $CH_3$ | $CH_3$ | Smp.88-92° (Isomeres B) |
| 1.6c | 2-Cl | 4-Cl | H | $CH_3$ | $CH_3$ | Smp. 85-87° (Isomeres C) |

Tabelle 1 (Fortsetzung)

| Verb. Nr. | $R'_a$ | $R'_b$ | $R'_c$ | $R_1$ | $R_2$ | Physikal-Konstante |
|---|---|---|---|---|---|---|
| 1.6a,b | 2-Cl | 4-Cl | H | $CH_3$ | $CH_3$ | Smp.96-101° (Isomere A+B) |
| 1.7a | 2-Cl | 4-Cl | H | $CH_2OCH_3$ | H | $n_D^{40}$ 1,5619 (Isomeres A) |
| 1.7b | 2-Cl | 4-Cl | H | $CH_2OCH_3$ | H | $n_D^{40}$ 1,5552 (Isomeres B) |
| 1.7a,b | 2-Cl | 4-Cl | H | $CH_2OCH_3$ | H | $n_D^{40}$ 1,5615 (Isomere A+B) |
| 1.8a | 2-Cl | 4-Cl | H | $CH_2Cl$ | H | $n_D^{50}$ 1,5763 (Isomeres A) |
| 1.8b | 2-Cl | 4-Cl | H | $CH_2Cl$ | H | $n_D^{50}$ 1,5783 (Isomeres B) |
| 1.8a,b | 2-Cl | 4-Cl | H | $CH_2Cl$ | H | $n_D^{50}$ 1,5746 (Isomere A+B) |
| 1.9 | 2-Cl | 4-Br | H | H | H | |
| 1.10 | 2-Cl | 4-Br | H | $CH_3$ | H | |
| 1.11a | 2-Cl | 4-Br | H | $C_2H_5$ | H | $n_D^{50}$ 1,5510 (Isomeres A) |
| 1.11b | 2-Cl | 4-Br | H | $C_2H_5$ | H | $n_D^{50}$ 1,5641 (Isomeres B) |
| 1.11a,b | 2-Cl | 4-Br | H | $C_2H_5$ | H | $n_D^{50}$ 1,5481 (Isomere A+B) |
| 1.12 | 2-Cl | 4-Br | H | $C_3H_7(n)$ | H | $n_D^{50}$ 1,5408 |
| 1.13 | 2-Cl | 4-Br | H | $CH_3$ | $CH_3$ | Smp.108-111° |
| 1.14 | 2-Cl | 4-Br | H | $CH_2OCH_3$ | H | $n_D^{50}$ 1,5638 |
| 1.15 | 2-Cl | 4-Br | H | $CH_2Cl$ | H | $n_D^{50}$ 1,5767 |
| 1.16 | 2-Cl | 4-F | H | H | H | $n^{50}$ 1,5691 |

Tabelle 1 (Fortsetzung)

| Verb. Nr. | $R'_a$ | $R'_b$ | $R'_c$ | $R_1$ | $R_2$ | Physikal-Konstante |
|---|---|---|---|---|---|---|
| 1.17 | 2-Cl | 4-F | H | $CH_3$ | H | |
| 1.18 | 2-Cl | 4-F | H | $C_2H_5$ | H | $n_D^{50}$ 1,5610 |
| 1.19 | 2-Cl | 4-F | H | $C_3H_7(n)$ | H | $n_D^{50}$ 1,5573 |
| 1.20 | 2-$OCHF_2$ | 4-Cl | H | $CH_3$ | H | |
| 1.21 | 2-$OCHF_2$ | 4-Cl | H | $C_2H_5$ | H | |
| 1.22 | 2-$OCHF_2$ | 4-Cl | H | $C_3H_7(n)$ | H | viscos |
| 1.23 | 2-$OCF_3$ | 4-Cl | H | $C_2H_5$ | H | |
| 1.24 | 2-$OCF_3$ | 4-Cl | H | $C_3H_7(n)$ | H | viscos |
| 1.25 | 2-$NO_2$ | 4-$NO_2$ | H | $C_2H_5$ | H | |
| 1.26 | 2-$NO_2$ | 4-$NO_2$ | H | $C_3H_7(n)$ | H | |
| 1.27 | H | 4-Cl | H | $C_3H_7(n)$ | H | |
| 1.28 | 3-Cl | 4-Cl | H | $C_3H_7(n)$ | H | |
| 1.29 | 2-$CH_3$ | 4-Cl | H | $C_2H_5$ | H | |
| 1.30 | 2-Cl | 4-Cl | 6-Cl | $C_2H_5$ | H | |
| 1.31 | 2-$OCH_3$ | 4-Cl | H | $C_3H_7(n)$ | H | |
| 1.32 | 2-CN | 4-Cl | H | $C_3H_7(n)$ | H | |
| 1.33 | 4-O-Ph-Cl(4) | H | H | H | H | |
| 1.34 | 4-O-Ph-Cl(4) | H | H | $CH_3$ | $CH_3$ | Smp.122-125° |
| 1.35 | 4-O-Ph-Cl(4) | H | H | $CH_3$ | H | |
| 1.36 | 4-O-Ph-Cl(4) | H | H | $C_2H_5$ | H | $n_D^{50}$ 1,5826 |
| 1.37 | 4-O-Ph-Cl(4) | H | H | $C_3H_7(n)$ | H | $n_D^{50}$ 1,5793 |
| 1.38 | 4-O-Ph-Cl(4) | 2-Cl | H | $C_2H_5$ | H | |
| 1.39 | 4-O-Ph-Cl(4) | 2-Cl | H | $CH_3$ | H | |
| 1.40 | 4-O-Ph-Cl(4) | 2-Cl | H | $C_3H_7(n)$ | H | $n_D^{50}$ 1,5613 |
| 1.41 | 4-O-Ph-Cl(4) | 2-$CH_3$ | H | $CH_3$ | H | |
| 1.42 | 4-O-Ph-Cl(4) | 2-$CH_3$ | H | $C_2H_5$ | H | $n_D^{50}$ 1,5664 |
| 1.43 | 4-O-Ph-Cl(4) | 2-$CH_3$ | H | $C_3H_7(n)$ | H | |
| 1.44 | 4-O-Ph-Cl(4) | 2-$CH_3$ | H | $CH_2OCH_3$ | H | $n_D^{50}$ 1,5784 |

Tabelle 1 (Fortsetzung)

| Verb. Nr. | $R'_a$ | $R'_b$ | $R'_c$ | $R_1$ | $R_2$ | Physikal-Konstante |
|---|---|---|---|---|---|---|
| 1.45 | 4-O-Ph-Cl(4) | 2-Cl | H | $CH_2OCH_3$ | H | $n_D^{50}$ 1,5743 |
| 1.46 | 4-O-Ph-Br(4) | H | H | $C_2H_5$ | H | |
| 1.47 | 4-O-Ph-Br(4) | 2-Cl | H | $C_2H_5$ | H | |
| 1.48 | 4-O-Ph-Br(4) | 2-Cl | H | $CH_3$ | H | $n_D^{50}$ 1,5850 |
| 1.49 | 4-O-Ph-Br(4) | 2-Cl | H | $C_3H_7$(n) | H | |
| 1.50 | 4-O-Ph-Br(4) | 2-$CH_3$ | H | $C_2H_5$ | H | |
| 1.51 | 4-O-Ph-Br(4) | 2-$CH_3$ | H | $CH_3$ | H | |
| 1.52 | 4-S-Ph-Cl(4) | H | H | $CH_3$ | H | |
| 1.53 | 4-S-Ph-Cl(4) | H | H | $C_2H_5$ | H | |
| 1.54 | 4-S-Ph-Cl(4) | 2-Cl | H | $CH_3$ | H | dunkles Oel |
| 1.55 | 4-S-Ph-Cl(4) | 2-Cl | H | $C_2H_5$ | H | |
| 1.56 | 4-S-Ph-Cl(4) | 2-$CH_3$ | H | $CH_3$ | H | |
| 1.57 | 4-S-Ph-Cl(4) | 2-$CH_3$ | H | $C_2H_5$ | H | viscos |
| 1.58 | 4-Ph | H | H | $C_2H_5$ | H | $n_D^{40}$ 1,5733 |
| 1.59 | 4-$OCH_3$ | | H | $C_2H_5$ | H | |
| 1.60 | 2-F | 4-Cl | H | H | H | |
| 1.61 | 2-F | 4-Cl | H | $CH_3$ | H | |
| 1.62a | 2-F | 4-Cl | H | $CH_3$ | $CH_3$ | $n_D^{50}$ 1,5352 (Isomeres A) |
| 1.62b | 2-F | 4-Cl | H | $CH_3$ | $CH_3$ | $n_D^{50}$ 1,5338 (Isomeres B) |
| 1.62a,b | 2-F | 4-Cl | H | $CH_3$ | $CH_3$ | $n_D^{50}$ 1,5304 (Isomere A+B) |
| 1.63a | 2-F | 4-Cl | H | $C_2G_5$ | H | $n_D^{50}$ 1,5331 (Isomeres A) |

Tabelle 1 (Fortsetzung)

| Verb. Nr. | $R'_a$ | $R'_b$ | $R'_c$ | $R_1$ | $R_2$ | Physikal-Konstante |
|---|---|---|---|---|---|---|
| 1.63b | 2-F | 4-Cl | H | $C_2H_5$ | H | $n_D^{50}$ 1,5350 (Isomeres B) |
| 1.63a,b | 2-F | 4-Cl | H | $C_2H_5$ | H | $n_D^{50}$ 1,5505 (Isomere A+B) |
| 1.64a | 2-F | 4-Cl | H | $C_3H_7(n)$ | H | $n_D^{50}$ 1,5269 (Isomeres A) |
| 1.64b | 2-F | 4-Cl | H | $C_3H_7(n)$ | H | $n_D^{50}$ 1,5291 (Isomeres B) |
| 1.64a,b | 2-F | 4-Cl | H | $C_3H_7(n)$ | H | $n_D^{50}$ 1,5293 (Isomere A+B) |
| 1.65 | 2-F | 4-Cl | H | $CH_2OCH_3$ | H | |
| 1.66 | H | 4-Cl | H | H | H | $n_D^{40}$ 1,5823 |

Tabelle 2: Verbindungen der Formel

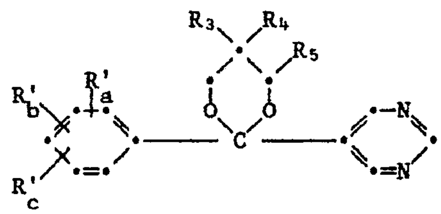

| Verb. Nr. | $R'_a$ | $R'_b$ | $R'_c$ | $R_3$ | $R_4$ | $R_5$ | Physikal. Konstante |
|---|---|---|---|---|---|---|---|
| 2.1 | 2-Cl | 4-Cl | H | H | H | H | Smp. 99-102° |
| 2.2 | 2-Cl | 4-Cl | H | $CH_3$ | $CH_3$ | H | Smp.108-111° |
| 2.3 | 2-Cl | 4-Cl | H | $CH_3$ | $CH_3$ | $CH_3$ | $n_D^{50}$ 1,5486 |
| 2.4 | 2-Cl | 4-Cl | 6-Cl | $CH_3$ | $CH_3$ | H | Smp.123-125° |
| 2.5 | 2-Cl | 4-Br | H | $CH_3$ | $CH_3$ | H | |
| 2.6 | 2-Cl | 4-F | H | $CH_3$ | $CH_3$ | H | |
| 2.7 | 4-O-Ph-Cl(4) | H | H | $CH_3$ | $CH_3$ | H | Smp. 63-66° |
| 2.8 | 4-O-Ph-Cl(4) | 2-Cl | H | $CH_3$ | $CH_3$ | H | |
| 2.9 | 4-O-Ph-Cl(4) | 2-$CH_3$ | H | $CH_3$ | $CH_3$ | H | Smp. 68-71° |
| 2.10 | 4-O-Ph-(4) | 2-Cl | H | $CH_3$ | $CH_3$ | H | |
| 2.11 | 4-O-Ph-Br(4) | 2-$CH_3$ | H | $CH_3$ | $CH_3$ | H | |
| 2.12 | 4-Ph | H | H | $CH_3$ | $CH_3$ | H | Smp.183-185° |
| 2.13 | 4-S-Ph-Cl(4) | H | H | H | H | H | |
| 2.14 | 4-S-Ph-Cl(4) | 2-Cl | H | $CH_3$ | $CH_3$ | H | viscos |
| 2.15 | 4-S-Ph-Cl(4) | 2-$CH_3$ | H | $CH_3$ | $CH_3$ | H | Oel |
| 2.16 | 4-O-Ph-Cl(4) | 2-$CH_3$ | H | $CH_3$ | $CH_3$ | $CH_3$ | |
| 2.17 | 2-F | 4-Cl | H | $CH_3$ | $CH_3$ | H | Smp. 85-93° |
| 2.18 | H | 4-Cl | H | $CH_3$ | $CH_3$ | H | |
| 2.19a,b | 2-Cl | 4-Cl | H | $C_2H_5$ | $CH_3$ | H | $n_D^{50}$ 1,5510 (Isomere A+B) |
| 2.19a | 2-Cl | 4-Cl | H | $C_2H_5$ | $CH_3$ | H | $n_D^{50}$ 1,5518 (Isomeres A) |
| 2.19b | 2-Cl | 4-Cl | H | $C_2H_5$ | $CH_3$ | H | $n_D^{50}$ 1,5527 (Isomeres B) |
| 2.20 | 2-Cl | 4-Cl | H | $C_2H_5$ | $C_2H_5$ | H | $n_D^{50}$ 1,5548 |

Tabelle 2 (Fortsetzung)

| Verb. Nr. | $R'_a$ | $R'_b$ | $R'_c$ | $R_3$ | $R_4$ | $R_5$ | Physikal. Konstante |
|---|---|---|---|---|---|---|---|
| 2.21 | H | 4-$NO_2$ | H | $CH_3$ | $CH_3$ | H | Smp. 118-125° |
| 2.22 | H | 4-Cl | H | $CH_3$ | $CH_3$ | H | Smp. 107-110° |
| 2.23a,b | 2-CN | 4-Cl | H | $C_2H_5$ | $CH_3$ | H | $n_D^{50}$ 1,5537 |
| 2.24a,b | 4-O-Ph-Cl(4) | 2-Cl | H | H | H | $C_2H_5$ | Oel |
| 2.25a,b | 4-O-Ph-Cl(4) | 2-$CH_3$ | H | H | H | $C_2H_5$ | Oel |

Tabelle 3

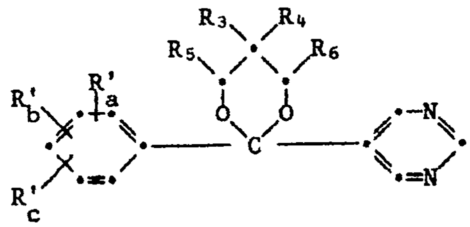

| Verb. Nr. | $R'_a$ | $R'_b$ | $R'_c$ | $R_3$ | $R_4$ | $R_5$ | $R_6$ | Physikal. Konstante |
|---|---|---|---|---|---|---|---|---|
| 3.1 | 2-Cl | 4-Cl | H | H | H | $CH_3$ | $CH_3$ | $n_D^{50}$ 1,5430 |
| 3.2 | 2-Cl | 4-Cl | H | H | $CH_3$ | $CH_3$ | $CH_3$ | $n_D^{50}$ 1,5486 |
| 3.3a | 2-Cl | 4-Cl | H | $C_2H_5$ | $CH_2OH$ | H | H | $n_D^{50}$ 1,5489 (Isomeres A) |
| 3.3b | 2-Cl | 4-Cl | H | $C_2H_5$ | $CH_2OH$ | H | H | Smp. 138-145° |
| 3.3a,b | 2-Cl | 4-Cl | H | $C_2H_5$ | $CH_2OH$ | H | H | |
| 3.4 | 2-Cl | 4-Cl | H | $CH_3$ | $CH_2OH$ | H | H | Smp. 154-160° |
| 3.5a | 2-Cl | 4-Cl | H | $CH_3$ | $CH_3$ | H | $C_3H_7(i)$ | Smp. 106-109° |
| 3.5b | 2-Cl | 4-Cl | H | $CH_3$ | $CH_3$ | H | $C_3H_7(i)$ | |
| 3.5a,b | 2-Cl | 4-Cl | H | $CH_3$ | $CH_3$ | H | $C_3H_7(i)$ | $n_D^{50}$ 1,5438 |
| 3.6a,b | 2-Cl | 4-Cl | H | H | H | H | $CH_3$ | $n_D^{50}$ 1,5476 |
| 3.7a,b | 2-Cl | 4-F | H | H | H | H | $CH_3$ | dunkles Oel |
| 3.8a,b | 2-F | 4-Cl | H | H | H | H | $CH_3$ | $n_D^{50}$ 1,5533 |
| 3.9a,b | 2-CN | 4-Cl | H | H | H | H | $CH_3$ | $n_D^{50}$ 1,5490 |
| 3.10a,b | 2-CN | 4-Cl | H | H | H | H | $C_2H_5$ | $n_D^{50}$ 1,5424 |
| 3.11a,b | 2-Cl | 4-Cl | H | H | H | H | $C_2H_5$ | $n_D^{50}$ 1,5411 |
| 3.12a,b | 2-F | 4-Cl | H | H | H | H | $C_2H_5$ | $n_D^{50}$ 1,5473 |

Tabelle 0: Verbindungen der Formel

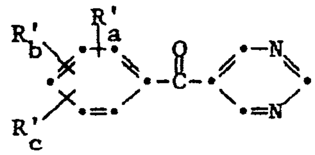

| Verb. Nr. | $R'_a$ | $R'_b$ | $R'_c$ | Physikal. Konstante |
|---|---|---|---|---|
| 0.1 | 2-Cl | 4-Cl | H | Smp. 75-78° |
| 0.2 | 2-Cl | 4-F | H | Smp. 64-66° |
| 0.3 | 2-Cl | 4-Br | H | Smp. 79-82° |
| 0.4 | H | 4-Cl | H | Smp.127-128° |
| 0.5 | H | 4-O-Ph | H | Smp.120-123° |
| 0.6 | H | 4-O-Ph-Cl(4) | H | Smp.114-116° |
| 0.7 | H | 4-O-Ph-Br(4) | H | Smp.123-126° |
| 0.8 | 2-Cl | 4-O-Ph-Br(4) | H | Smp.112-116° |
| 0.9 | 2-F | 4-Cl | H | Smp. 92-96° |
| 0.10 | H | 4-$NO_2$ | H | Smp.122-124° |
| 0.11 | 2-Cl | 4-O-Ph-Cl(4) | H | Smp.108-111° |
| 0.12 | 2-$OCHF_2$ | 4-Cl | H | Smp. 65-68° |
| 0.13 | 2-$OCF_3$ | 4-Cl | H | Smp. 66-68° |
| 0.14 | 2-CN | 4-Cl | H | Smp. 58-61° |
| 0.15 | 2-$CH_3$ | 4-O-Ph-Cl(4) | H | Smp.103-105° |
| 0.16 | 2-Cl | 4-Cl | 6-Cl | Smp. 92-96° |
| 0.17 | 2-Cl | 4-S-Ph-Cl(4) | H | Smp. 82-87° |

## 2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen 1-3 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen 1-3 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3 Granulate | a) | b) |
|---|---|---|
| Wirkstoff aus den Tabellen 1-3 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen 1-3 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

**Formulierungsbeispiele für feste Wirkstoffe der Formel I**

**(% = Gewichtsprozent)**

| 2.5 Spritzpulver | a) | b) | c) |
|---|---|---|---|
| Wirkstoff aus den Tabellen 1-3 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen 1-3 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen 1-3 | 5 % | 8 % |
| Talkum | 95 % | - |
| Kaolin | - | 92 % |

Man erhält anwendungsfertige Stäubemittel, indem der Wirkstoff mit den Träger vermischt und auf einer geeigneten Mühle vermahlen wird

2.8 Extruder Granulat

| | |
|---|---|
| Wirkstoff aus den Tabellen 1-3 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.9 Umhüllungs-Granulat

| | |
|---|---|
| Wirkstoff aus den Tabellen 1-3 | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.10 Suspensions-Konzentrat

| | |
|---|---|
| Wirkstoff aus den Tabellen 1-3 | 40 % |
| Ethylenglykol | 10 % |
| Nonylphenolpolyethylenglykolether (15 Mol Ethylenoxid) | 6 % |
| N-Lingninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele:

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C

aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einem Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Verbindungen aus den Tabellen zeigen gegen Puccinia-Pilze gute Wirksamkeit. Unbehandelte aber infizierte Kontrollpflanzen weisen dagegen einen Puccinia-Befall von 100 % auf. Nach Behandlung mit den Verbindungen Nr. 1.3a; 1.3b; 1.4a; 1.4a,b; 1.5; 1.7a; 1.7b; 1.7a,b; 1.6a; 1.6b; 1.6a,b; 1.22; 1.24; 1.34; 1.36; 1.37; 1.40; 1.42; 1.44; 1.45; 1.54; 1.58; 2.2; 2.19a,b; 2.20; 2.7; 2.9; 2.12; 2.14; 2.15; 2.23a,b; 2.24a,b; 2.25a,b; 3.6a,b; 3.8a,b; 3.10a,b; 3.11a,b; 3.12a,b beträgt der Puccinia-Befall 5-20 % oder weniger.

Beispiel 3.2: Wirkung gegen Cercospora archidicola auf Erdnusspflanzen

Residual-protektive Wirkung

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen behandelt wurden, einen stark reduzierten Cercospora-Befall. So verhindern z.B. die Verbindungen Nr. 1.3a; 1.4b; 1.4a,b; 1.6a; 1.6b; 1.6a,b; 1.34; 1.37; 1.44; 1.45; 1.54; 2.7; 2.9; 2.19a,b; 2.20; 2.23a,b; 3.6a,b; 3.8a,b; 3.11a,b; 3.12a,b im obigen Versuch das Auftreten von Flecken fast vollständig (0-10 %).

Beispiel 3.3: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen aus den Tabellen zeigen gute Wirksamkeit gegen Erysiphe-Pilze. Unbehandelte, aber infizierte Kontrollpflanzen weisen dagegen einen Erysiphe-Befall von 100 % auf. So hemmen z.B. die Verbindungen Nr. 1.1; 1.3a; 1.3b; 1.3a,b; 1.4a; 1.4b; 1.4a,b; 1.5; 1.6a; 1.6b; 1.6a,b; 1.7a; 1.7b; 1.7a,b; 1.22; 1.24; 1.34,; 1.37; 1.40; 1.42,; 1.44; 1.45; 1.54; 1.58; 2.2; 2.7; 2.9; 2.12; 2.14; 2.15; 2.19a,b; 2.19a; 2.23a,b; 2.24a,b; 2.25a,b; 3.6a,b; 3.8a,b; 3.9a,b; 3.10a,b; 3.11a,b; 3.12a,b den Pilzbefall auf 0-5 %.

Beispiel 3.4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,06 % Aktivsubstanz) besprüht. Nach 24 Stunden werden die behandelten

Pflanzen mit einer Konidiensuspension das Pilzes infiziert. Die Pflanzen werden dann während 5 Tagen bei 90-100 % relativer Luftfeuchtigkeit inkubiert und während 10 weiteren Tagen in einem Gewächshaus bei 20-24 °C aufgestellt. Der Schorfbefall wird 15 Tage nach der Infektion beurteilt. Verbindungen aus den Tabellen hemmen den Krankheitsbefall bis auf weniger als 10 %. Unbehandelte jedoch infizierte Triebe zeigen dagegen einen 100%igen Venturia-Befall. So hemmen z.B. die Verbindungen Nr. 1.3a; 1.34; 1.40; 1.42; 2.19a,b; 2.23a,b; 2.24a,b; 2.25a,b; 3.6a,b; 3.8a,b; 3.9a,b; 3.10a,b; 3.11a,b; 3.12a,b den Pilzbefall auf Apfeltrieben auf 0 bis 5 %.

Beispiel 3.5: Wirkung gegen Botrytis cinerea auf Bohnen Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21 °C erfolgt die Beurteilung des Pilzbefalls. Verbindungen aus den Tabellen hemmen die Pilzinfektion sehr stark. So vermindern z.B. die Verbindungen Nr. 1.3a; 1.3b; 1.3a,b; 1.4a; 1.4b; 1.4a,b; 1.6a; 1.6b; 1.6a,b; 1.7a; 1.7b; 1.7a,b und 2.2; 2.12; 2.24a,b; 2.25a,b; 3.6a,b; 3.11a,b den Pilzbefall auf 0 bis 5 %.

Beispiel 3.6: Wirkung gegen Pyricularia oryzae auf Reispflanzen

a) Residual-protektive Wirkung

Reispflanzen werden nach zweiwöchiger Anzucht mit einer aus Spritzpulver des Wirkstoffes hergestellten Spritzbrühe (0,05 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24 °C wird der Pilzbefall beurteilt.

b) Systemische Wirkung

Zu zwei Wochen alten in für Blumen üblichen Tontöpfen wachsenden Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 48 Stunden werden die behandelten Reispflanzen mit einer Konidiensuspension des Pilzes infiziert. Nach einer Inkubation der infizierten Pflanzen während 5 Tagen bei 95-100 % relativer Luftfeuchtigkeit und ca. 24 ° wird der Pilzbefall beurteilt.

Verbindungen aus den Tabellen zeigen gute Wirksamkeit gegen den Pyriculariapilz. Unbehandelte, jedoch infizierte Kontrollpflanzen weisen dagegen einen Pyricularia-Befall von 100 % auf. So hemmen z.B. die Verbindungen 1.3a; 1.3b; 1.4a und 1.6a den Pilzbefall bis auf 0 bis 5 %.

Beispiel 3.7: Wirkung gegen Rhizoctonia solani (Bodenpilz auf Reispflanzen

a) Protektiv-lokale Bodenapplikation

12 Tage alte Reispflanzen werden mit einer aus einer Formulierung des Wirkstoffes hergestellten Spritzbrühe (0,006 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27 °C (Tag), bzw. 23 °C (Nacht) und 100 % rel. Luftfeuchtigkeit (Feuchtkasten) in der Klimakammer wird der Pilzbefall auf Blattscheide, Blättern und Stengel beurteilt.

b) Protektiv-lokale Blattapplikation

12 Tage alte Reispflanzen werden mit einer aus einer Formulierung der Wirkstoffe hergestellten Spritzbrühe besprüht. Ein Tag später werden die behandelten Pflanzen mit einer Suspension von Myzel und Sklerotien von R. solani infiziert. Nach 6-tägiger Inkubation bei 27 °C (Tag), bzw. 23 °C (Nacht) und 100 % rel. Luftfeuchtigkeit (Feuchtkasten) in der Klimakammer wird dar Pilzbefall auf Blattscheide, Blättern und Stengel beurteilt.

Verbindungen aus den Tabellen zeigen gute Wirksamkeit durch Hemmung des Rhizoctonia-Befalls.

Unbehandelte jedoch infizierte Kontrollpflanzen weisen dagegen einen Befall von 100 % auf. So hemmen z.B. die Verbindungen 1.3a; 1.3b; 1.4a; 1.4a,b; 1.6a; 1.6b; 1.6a,b; 1.7a; 1.7b; 1.7a,b und 2.2 den Pilzbefall bis auf 0 bis 5 %.

**Patentansprüche**

1. Verbindungen der allgemeinen Formel I

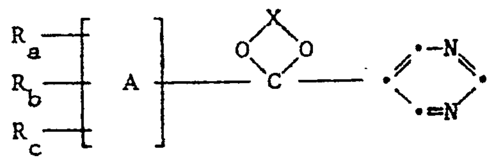 (I) ,

in welcher
A für Phenyl, Phenoxyphenyl, Phenylthiophenyl, Biphenyl oder Naphthyl steht,
$R_a$, $R_b$ und $R_c$ unabhängig voneinander Wasserstoff, Halogen, Cyano, Nitro, $C_1$-$C_4$-Alkyl, $C_1$-$C_4$-Alkoxy, $C_1$-$C_4$-Haloalkyl oder $C_1$-$C_4$-Haloalkoxy darstellt, und
X eines der folgenden Brückenelemente

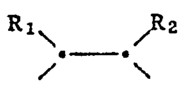 oder 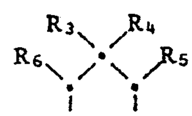

bedeutet,
worin $R_1$ und $R_2$ unabhängig voneinander Wasserstoff, $C_1$-$C_4$-Alkyl, $C_1$-$C_4$-Haloalkyl oder $CH_2O$-$C_1$-$C_4$-Alkyl darstellen und
$R_4$ Wasserstoff, $C_1$-$C_4$-Alkyl oder $CH_2OH$ und
$R_3$, $R_5$, und $R_6$ unabhängig voneinander Wasserstoff oder $C_1$-$C_4$-Alkyl bedeuten, einschliesslich ihrer Säureadditionssalze.

2. Verbindungen der Formel I nach Anspruch 1, worin A, $R_a$, $R_b$, $R_c$, $R_1$ und $R_2$ die genannte Bedeutung haben, während $R_6$ Wasserstoff ist und $R_3$, $R_4$ und $R_5$ unabhängig voneinander Wasserstoff oder $C_1$-$C_4$-Alkyl bedeuten.

3. Verbindungen der Formel I nach Anspruch 1, worin A für Phenyl, Phenoxyphenyl oder Phenylthiophenyl steht, $R_a$, $R_b$ und $R_c$ unabhängig voneinander Wasserstoff, Halogen, $C_1$-$C_2$-Alkyl, $C_1$-$C_2$-Alkoxy, $C_1$-$C_2$-Haloalkyl, $C_1$-$C_2$-Haloalkoxy, Nitro oder Cyano darstellen, X eines der Brückenelemente

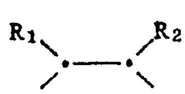 oder 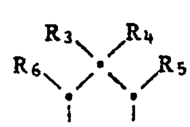

bedeutet,
worin $R_1$ und $R_2$ unabhängig voneinander Wasserstoff, $C_1$-$C_4$-Alkyl, $C_1$-$C_2$-Haloalkyl oder $CH_2O$-$C_1$-$C_2$-Alkyl darstellen und $R_4$ Wasserstoff, $C_1$-$C_2$-Alkyl oder $CH_2OH$ und $R_3$, $R_5$ und $R_6$ unabhängig voneinander Wasserstoff oder $C_1$-$C_2$-Alkyl bedeuten.

4. Verbindungen der Formel I nach Anspruch 2, worin A für Phenyl, Phenoxyphenyl oder Phenylthiophenyl steht, $R_a$, $R_b$ und $R_c$ unabhängig voneinander Wasserstoff, Halogen, $C_1$-$C_2$-Alkyl, $C_1$-$C_2$-Alkoxy, $C_1$-$C_2$-Haloalkyl, $C_1$-$C_2$-Haloalkoxy, Nitro oder Cyano darstellen, X eines der Brückenelemente

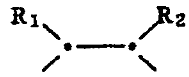 oder 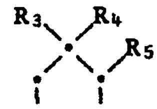

bedeutet,
$R_1$ und $R_2$ unabhängig voneinander Wasserstoff, $C_1$-$C_4$-Alkyl, $C_1$-$C_2$-Haloalkyl oder $CH_2$-O-($C_1$-$C_2$)-Alkyl sind, und $R_3$, $R_4$ und $R_5$ unabhängig voneinander Wasserstoff oder $C_1$-$C_2$-Alkyl darstellen.

5. Verbindungen dar Formel I nach Anspruch 3, worin A für Phenyl oder Phenoxyphenyl steht, $R_a$, $R_b$ und $R_c$ unabhängig voneinander Wasserstoff, Halogen, $C_1$-$C_2$-Alkyl, $C_1$-$C_2$-Alkoxy, $C_1$-$C_2$-Haloalkoxy, Nitro oder Cyano darstellen, $R_1$ und $R_2$ unabhängig voneinander Wasserstoff, $C_1$-$C_4$-Alkyl oder $CH_2OCH_3$ darstellen und $R_4$ Wasserstoff, Methyl oder $CH_2OH$ und $R_3$, $R_5$ und $R_6$ unabhängig voneinander Wasserstoff oder Methyl bedeuten.

6. Verbindungen der Formel I nach Anspruch 5, worin A für Phenyl oder Phenoxyphenyl steht, $R_a$, $R_b$ und $R_c$ unabhängig voneinander Cl und/oder Methyl, unter Besetzung der 2- und/oder 4-Stellung bedeuten, und
X das Brückenelement

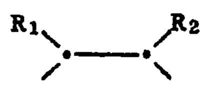

darstellt,
worin $R_1$ $C_1$-$C_3$-Alkyl oder $CH_2OCH_3$ und $R_2$ Wasserstoff bedeuten.

7. Verbindungen der Formel I nach Anspruch 4, worin A für Phenyl oder Phenoxyphenyl steht; $R_a$, $R_b$ und $R_c$ unabhängig voneinander Chlor oder Methyl unter Besetzung der 2- und/oder 4-Stellung bedeuten, und X das Brückenelement

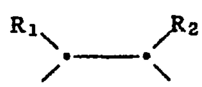

darstellt,
worin $R_1$ $C_1$-$C_3$-Alkyl oder $CH_2OCH_3$ und $R_2$ Wasserstoff sind.

8. Verbindungen der Formel I nach Anspruch 1, worin A in Kombination mit $R_a$, $R_b$ und $R_c$ 2,4-Dichlorphenyl, 2-Fluor-4-chlorphenyl, 2-Cyano-4-chlorphenyl, 2-Methyl-4-chlorphenyl, p-Chlorphenoxy, 2-Methyl-4-(p-chlorphenoxy) oder 2-Chlor-4-(p-chlorphenoxy) bedeutet, während $R_1$ bis $R_6$ Wasserstoff oder $C_1$-$C_4$-Alkyl darstellen, zusammen aber höchstens 5 C-Atome besitzen.

9. Ein Isomeres oder ein Isomerengemisch der Verbindungen 5-[2-(2',4'-Dichlorphenyl)-4-ethyl-1,3-dioxolan-2-yl]-pyrimidin,
5-[2-(2',4'-Dichlorphenyl)-4-propyl-1,3-dioxolan-2-yl]-pyrimidin,
5-[2-(2',4'- Dichlorphenyl)-4-methoxymethyl-1,3-dioxolan-2-yl]pyrimidin,
5-[2-(2',4'-Dichlorphenyl)-4, 5-dimethyl-1,3-dioxolan-2-yl]pyrimidin,
5-[2-(2',4'-Dichlorphenyl)-5,5-dimethyl-1,3-dioxan-2-yl]pyrimidin,

10. Ein Isomeres oder ein Isomerengemisch der Verbindungen 5-[2-(2',4'-Dichlorphenyl)-4,6-dimethyl-1,3-dioxan-2-yl]pyrimidin,
5-[2-(2',4'-Dichlorphenyl)-4,5,6-trimethyl-1,3-dioxan-2-yl]pyrimidin,
5-[2-(2',4'-Dichlorphenyl)-5-ethyl-5-methyl-1,3-dioxan-2yl]-pyrimidin,
5-[2-(2',4'-Dichlorphenyl)-4-methyl-1,3-dioxan-2yl]-pyrimidin,

5-[2-(2'-Cyano-4'-chlorphenyl)-5-ethyl-5-methyl-1,3-dioxan-2yl]-pyrimidin,
5-[2-(2',4'-Dichlorphenyl)-4-ethyl-1,3-dioxan-2yl]-pyrimidin,
5-[2-(2'-Chlor-4'-p-chlorphenoxiphenyl)-4-ethyl-1,3-dioxan-2yl]pyrimidin.

**11.** Verfahren zur Herstellung von Verbindungen der Formel I nach Anspruch 1, gekennzeichnet,
A) durch die Ketalisierungsreaktion eines Ketons der Formel II

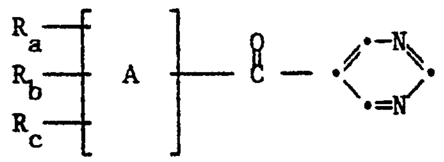 (II)

mit einem Diol der Formel III

HO-X-OH (III)

worin A, $R_a$, $R_b$, $R_c$ und X die unter Formel I angegebenen Bedeutungen besitzen, in einem inerten Lösungsmittel oder
B) durch Umsetzung eines Ketons der Formel IV

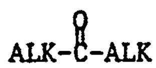 (IV)

mit einem Diol der Formel III in einem inerten Lösungsmittel zu einer Verbindung der Formel V

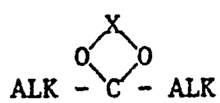 (V)

worin ALK ein $C_1$-$C_4$-Alkyl darstellt und X die unter Formel I angegebenen Bedeutungen besitzt, und anschliessende Ketalisierung eines Ketons der Formel II mit Hilfe der nicht aus dem Reaktionsgemisch isolierten Verbindung der Formel V in einem inerten Lösungsmittel.

**12.** Verfahren nach Anspruch 11, gekennzeichnet durch Zusatz einer Säure zur Ketalisierungsreaktion.

**13.** Mikrobizides Mittel zur Bekämpfung oder Verhütung eines Befalls von Pflanzen durch Mikroorganismen, dadurch gekennzeichnet, dass es als mindestens eine aktive Komponente eine Verbindung der Formel I gemäss Anspruch 1 enthält.

**14.** Mittel gemäss Anspruch 13, dadurch gekennzeichnet, dass es als aktive Komponente mindestens eine Verbindung der Formel I gemäss einem der Ansprüche 2, 4, 7 oder 9 enthält.

**15.** Mittel gemäss Anspruch 13, dadurch gekennzeichnet, dass es als aktive Komponente mindestens eine Verbindung der Formel I gemäss einem der Ansprüche 3, 5, 6, 8 oder 10 enthält.

**16.** Verfahren zur Bekämpfung oder Verhütung eines Befalls von Kulturpflanzen durch phytopathogene Mikroorganismen, dadurch gekennzeichnet, dass man eine Verbindung der Formel I gemäss Anspruch 1 auf die Pflanze oder ihren Standort oder auf Pflanzenteile appliziert.

**17.** Verfahren gemäss Anspruch 16, dadurch gekennzeichnet, dass man eine Verbindung der Formel I gemäss einem der Ansprüche 2, 4, 7 oder 9 appliziert.

**18.** Verfahren gemäss Anspruch 16, dadurch gekennzeichnet, dass man eine Verbindung der Formel I

gemäss einem der Ansprüche 3, 5, 6, 8 oder 10 appliziert.

19. Verfahren zur Herstellung eines agrochemischen Mittels, dadurch gekennzeichnet, dass man mindestens eine Verbindung der Formel I gemäss Anspruch 1 mit geeigneten festen oder flüssigen Zusatzstoffen und Tensiden innig vermischt.

**Patentansprüche für folgenden Vertragsstaat : AT**

1. Mikrobizides Mittel enthaltend neben üblichen Träger- und Hilfsstoffen mindestens eine Verbindung der allgemeinen Formel I

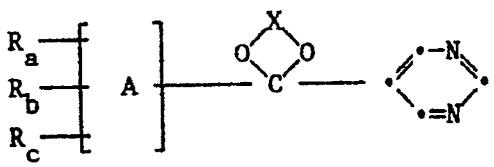
(I)

in welcher
A für Phenyl, Phenoxyphenyl, Phenylthiophenyl, Biphenyl oder Naphthyl steht,
$R_a$, $R_b$ und $R_c$ unabhängig voneinander Wasserstoff, Halogen, Cyano, Nitro, $C_1$-$C_4$-Alkyl, $C_1$-$C_4$-Alkoxy, $C_1$-$C_4$-Haloalkyl oder $C_1$-$C_4$-Haloalkoxy darstellt, und
X eines der folgenden Brückenelemente

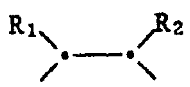
oder
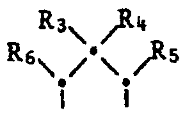

bedeutet,
worin $R_1$ und $R_2$ unabhängig voneinander Wasserstoff, $C_1$-$C_4$-Alkyl, $C_1$-$C_4$-Haloalkyl oder $CH_2O$-$C_1$-$C_4$-Alkyl darstellen und
$R_4$ Wasserstoff, $C_1$-$C_4$-Alkyl oder $CH_2OH$ und
$R_3$, $R_5$, und $R_6$ unabhängig voneinander Wasserstoff oder $C_1$-$C_4$-Alkyl bedeuten, einschliesslich ihrer Säureadditionssalze.

2. Mikrobizides Mittel enthaltend neben üblichen Träger- und Hilfsstoffen mindestens eine Verbindung der Formel I in Anspruch 1, worin A, $R_a$, $R_b$, $R_c$, $R_1$ und $R_2$ die genannte Bedeutung haben, während $R_6$ Wasserstoff ist und $R_3$, $R_4$ und $R_5$ unabhängig voneinander Wasserstoff oder $C_1$-$C_4$-Alkyl bedeuten.

3. Mittel nach Anspruch 1 enthaltend mindestens eine Verbindung der Formel I, worin A für Phenyl, Phenoxyphenyl oder Phenylthiophenyl steht, $R_a$, $R_b$ und $R_c$ unabhängig voneinander Wasserstoff, Halogen, $C_1$-$C_2$-Alkyl, $C_1$-$C_2$-Alkoxy, $C_1$-$C_2$-Haloalkyl, $C_1$-$C_2$-Haloalkoxy, Nitro oder Cyano darstellen, X eines der Brückenelemente

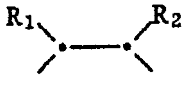
oder
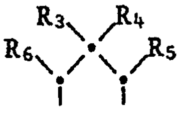

bedeutet,
worin $R_1$ und $R_2$ unabhängig voneinander Wasserstoff, $C_1$-$C_4$-Alkyl, $C_1$-$C_2$-Haloalkyl oder $CH_2O$-$C_1$-$C_2$-Alkyl darstellen und $R_4$ Wasserstoff, $C_1$-$C_2$-Alkyl oder $CH_2OH$ und $R_3$, $R_5$ und $R_6$ unabhängig voneinander Wasserstoff oder $C_1$-$C_2$-Alkyl bedeuten.

4. Mittel nach Anspruch 2 enthaltend mindestens eine Verbindung der Formel I in Anspruch 1, worin A für Phenyl, Phenoxyphenyl oder Phenylthiophenyl steht, $R_a$, $R_b$ und $R_c$ unabhängig voneinander Wasserstoff, Halogen, $C_1$-$C_2$-Alkyl, $C_1$-$C_2$-Alkoxy, $C_1$-$C_2$-Haloalkyl, $C_1$-$C_2$-Haloalkoxy, Nitro oder Cyano darstellen, X eines der Brückenelemente

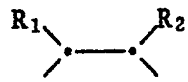
oder
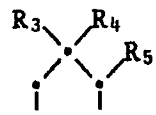

bedeutet,
$R_1$ und $R_2$ unabhängig voneinander Wasserstoff, $C_1$-$C_4$-Alkyl, $C_1$-$C_2$-Haloalkyl oder $CH_2$-O-($C_1$-$C_2$)-Alkyl sind, und $R_3$, $R_4$ und $R_5$ unabhängig voneinander Wasserstoff oder $C_1$-$C_2$-Alkyl darstellen.

5. Mittel nach Anspruch 3 enthaltend mindestens eine Verbindung der Formel I in Anspruch 1, worin A für Phenyl oder Phenoxyphenyl steht, $R_a$, $R_b$ und $R_c$ unabhängig voneinander Wasserstoff, Halogen, $C_1$-$C_2$-Alkyl, $C_1$-$C_2$-Alkoxy, $C_1$-$C_2$-Haloalkoxy, Nitro oder Cyano darstellen, $R_1$ und $R_2$ unabhängig voneinander Wasserstoff, $C_1$-$C_4$-Alkyl oder $CH_2OCH_3$ darstellen und $R_4$ Wasserstoff, Methyl oder $CH_2OH$ und $R_3$, $R_5$ und $R_6$ unabhängig voneinander Wasserstoff oder Methyl bedeuten.

6. Mittel nach Anspruch 5 enthaltend mindestens eine Verbindung der Formel I in Anspruch 1, worin A für Phenyl oder Phenoxyphenyl steht, $R_a$, $R_b$ und $R_c$ unabhängig voneinander Cl und/oder Methyl, unter Besetzung der 2- und/oder 4-Stellung bedeuten, und X das Brückenelement

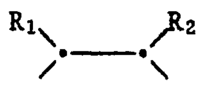

darstellt,
worin $R_1$ $C_1$-$C_3$-Alkyl oder $CH_2OCH_3$ und $R_2$ Wasserstoff bedeuten.

7. Mittel nach Anspruch 4 enthaltend mindestens eine Verbindung der Formel I in Anspruch 1, worin A für Phenyl oder Phenoxyphenyl steht; $R_a$, $R_b$ und $R_c$ unabhängig voneinander Chlor oder Methyl unter Besetzung der 2- und/oder 4-Stellung bedeuten, und X das Brückenelement

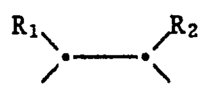

darstellt,
worin $R_1$ $C_1$-$C_3$-Alkyl oder $CH_2OCH_3$ und $R_2$ Wasserstoff sind.

8. Mittel nach Anspruch 6 enthaltend mindestens ein Mittel enthaltend mindestens eine Verbindung der Formel I in Anspruch 1, worin A in Kombination mit $R_a$, $R_b$ und $R_c$ 2,4-Dichlorphenyl, 2-Fluor-4-chlorphenyl,2-Cyano-4-chlorphenyl, 2-Methyl-4-chlorphenyl, p-Chlorphenoxy, 2-Methyl-4-(p-chlorphenoxy) oder 2-Chlor-4-(p-chlorphenoxy) bedeutet, während $R_1$ bis $R_6$ Wasserstoff oder $C_1$-$C_4$-Alkyl darstellen, zusammen aber höchstens 5 C-Atome besitzen.

9. Mittel nach Anspruch 2 enthaltend mindestens ein Isomeres oder ein Isomerengemisch der Verbindungen 5-[2-(2',4'-Dichlorphenyl)-4-ethyl-1,3-dioxolan-2-yl]-pyrimidin,
5-[2-(2',4'-Dichlorphenyl)-4-propyl-1,3-dioxolan-2-yl]-pyrimidin,
5-[2-(2',4'-Dichlorphenyl)-4-methoxmethyl-1,3-dioxolan-2-yl]-pyrimidin,
5-[2-(2',4'-Dichlorphenyl)-4,5-dimethyl-1,3-dioxolan-2-yl]pyrimidin,
5-[2-(2',4'-Dichlorphenyl)-5,5-dimethyl-1,3-dioxan-2-yl]pyrimidin,

10. Mittel nach Anspruch 1 enthaltend mindestens ein Isomeres oder ein Isomerengemisch der Verbindungen 5-[2-(2',4'-Dichlorphenyl)-4,6-dimethyl-1,3-dioxan-2-yl]pyrimidin,

5-[2-(2',4'-Dichlorphenyl)-4,5,6-trimethyl-1,3-dioxan-2-yl]pyrimidin,
5-[2-(2',4'-Dichlorphenyl)-5-ethyl-5-methyl-1,3-dioxan-2yl]-pyrimidin,
5-[2-(2',4'-Dichlorphenyl)-4-methyl-1,3-dioxan-2yl]-pyrimidin,
5-[2-(2'-Cyano-4'-chlorphenyl)-5-ethyl-5-methyl-1,3-dioxan-2yl]-pyrimidin,
5-[2-(2',4'-Dichlorphenyl)-4-ethyl-1,3-dioxan-2yl]-pyrimidin,
5-[2-(2'-Chlor-4'-p-chlorphenoxiphenyl)-4-ethyl-1,3-dioxan-2yl]-pyrimidin.

**11.** Verfahren zur Herstellung von Verbindungen der Formel I in Anspruch 1, gekennzeichnet,
A) durch die Ketalisierungsreaktion eines Ketons der Formel II

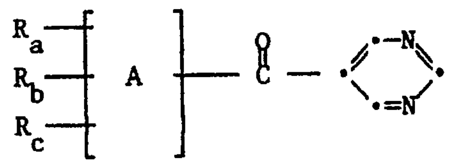

(II)

mit einem Diol der Formel III

HO-X-OH (III)

worin A, $R_a$, $R_b$, $R_c$ und X die unter Formel I angegebenen Bedeutungen besitzen, in einem inerten Lösungsmittel oder
B) durch Umsetzung eines Ketons der Formel IV

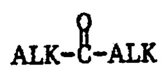

(IV)

mit einem Diol der Formel III in einem inerten Lösungsmittel zu einer Verbindung der Formel V

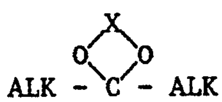

(V)

worin ALK ein $C_1$-$C_4$-Alkyl darstellt und X die unter Formel I angegebenen Bedeutungen besitzt, und anschliessende Ketalisierung eines Ketons der Formel II mit Hilfe der nicht aus dem Reaktionsgemisch isolierten Verbindung der Formel V in einem inerten Lösungsmittel.

**12.** Verfahren nach Anspruch 11, gekennzeichnet durch Zusatz einer Säure zur Ketalisierungsreaktion.

**13.** Mittel gemäss einem der Ansprüche 1-10 zur Bekämpfung oder Verhütung eines Befalls von Pflanzen durch Mikroorganismen.

**14.** Verfahren zur Bekämpfung oder Verhütung eines Befalls von Kulturpflanzen durch phytopathogene Mikroorganismen, dadurch gekennzeichnet, dass man eine gemäss Anspruch 1 definierte Verbindung der Formel I auf die Pflanze oder ihren Standort oder auf Pflanzenteile appliziert.

**15.** Verfahren gemäss Anspruch 14, dadurch gekennzeichnet, dass man eine gemäss einem der Ansprüche 2, 4, 7 oder 9 definierte Verbindung der Formel I appliziert.

**16.** Verfahren gemäss Anspruch 14, dadurch gekennzeichnet, dass man eine gemäss einem der Ansprüche 3, 5, 6, 8 oder 10 definierte Verbindung der Formel I appliziert.

**17.** Verwendung von gemäss Anspruch 11 hergestellten Verbindungen der Formel I zur Bekämpfung und/oder zur präventiven Verhütung eines Befalls von Pflanzen durch Mikroorganismen.

18. Verwendung nach Anspruch 17 von in einem der Ansprüche 2, 4, 7 oder 9 definierten Verbindungen der Formel I.

19. Verwendung nach Anspruch 17 von in einem der Ansprüche 3, 5, 6, 8 oder 10 definierten Verbindungen der Formel I.

20. Verwendung gemäss einem der Ansprüche 17 bis 19 dadurch gekennzeichnet, dass es sich bei den Mikroorganismen um phytophatogene Pilze handelt.

## Claims

1. A compound of the general formula I

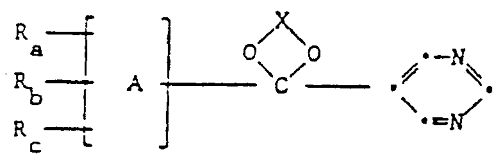 (I)

in which
A is phenyl, phenoxyphenyl, phenylthiophenyl, biphenyl or naphthyl,
$R_a$, $R_b$ and $R_c$ are each independently hydrogen, halogen, cyano, nitro, $C_1$-$C_4$alkyl, $C_1$-$C_4$alkoxy, $C_1$-$C_4$haloalkyl or $C_1$-$C_4$haloalkoxy and
X is one of the following bridge elements

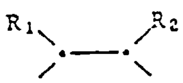 or 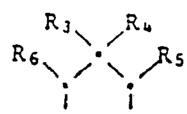

in which $R_1$ and $R_2$ are each independently hydrogen, $C_1$-$C_4$alkyl, $C_1$-$C_4$haloalkyl or $CH_2O$-$C_1$-$C_4$alkyl, $R_4$ is hydrogen, $C_1$-$C_4$alkyl or $CH_2OH$ and
$R_3$, $R_5$ and $R_6$ are each independently hydrogen or $C_1$-$C_4$alkyl, or an acid addition salt thereof.

2. A compound of formula I according to claim 1, in which A, $R_a$, $R_b$, $R_c$, $R_1$ and $R_2$ are as defined above and $R_6$ is hydrogen and $R_3$, $R_4$ and $R_5$ are each independently hydrogen or $C_1$-$C_4$alkyl.

3. A compound of formula I according to claim 1, in which A is phenyl, phenoxyphenyl, phenylthiophenyl, $R_a$, $R_b$ and $R_c$ are each independently hydrogen, halogen, $C_1$-$C_2$alkyl, $C_1$-$C_2$alkoxy, $C_1$-$C_2$haloalkyl, $C_1$-$C_2$haloalkoxy, nitro or cyano, X is one of the bridge elements

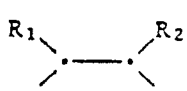 or 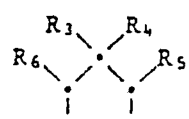

in which $R_1$ and $R_2$ are each independently hydrogen, $C_1$-$C_4$alkyl, $C_1$-$C_2$haloalkyl or $CH_2O$-$C_1$-$C_2$alkyl and $R_4$ is hydrogen, $C_1$-$C_2$alkyl or $CH_2OH$ and $R_3$, $R_5$ and $R_6$ are each independently hydrogen or $C_1$-$C_2$alkyl.

4. A compound of formula I according to claim 2, in which A is phenyl, phenoxyphenyl or phenylthiophenyl, $R_a$, $R_b$ and $R_c$ are each independently hydrogen, halogen, $C_1$-$C_2$alkyl, $C_1$-$C_2$alkoxy, $C_1$-$C_2$haloalkyl, $C_1$-$C_2$haloalkoxy, nitro or cyano, X is one of the bridge elements

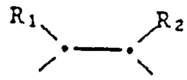

or

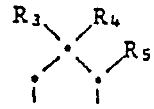

$R_1$ and $R_2$ are each independently hydrogen, $C_1$-$C_4$alkyl, $C_1$-$C_2$haloalkyl or $CH_2$-O-($C_1$-$C_2$)alkyl and $R_3$, $R_4$ and $R_5$ are each independently hydrogen or $C_1$-$C_2$alkyl.

5. A compound of formula I according to claim 3, in which A is phenyl or phenoxyphenyl, $R_a$, $R_b$ and $R_c$ are each independently hydrogen, halogen, $C_1$-$C_2$alkyl, $C_1$-$C_2$alkoxy, $C_1$-$C_2$haloalkoxy, nitro or cyano, $R_1$ and $R_2$ are each independently hydrogen, $C_1$-$C_4$alkyl or $CH_2OCH_3$ and $R_4$ is hydrogen, methyl or $CH_2OH$ and $R_3$, $R_5$ and $R_6$ are each independently hydrogen or methyl.

6. A compound of formula I according to claim 5, in which A is phenyl or phenoxyphenyl, $R_a$, $R_b$ and $R_c$ are each independently chlorine and/or methyl in the 2- and/or 4-position and X is the bridge element

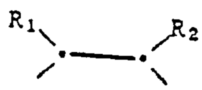

in which $R_1$ is $C_1$-$C_3$alkyl or $CH_2OCH_3$ and $R_2$ is hydrogen.

7. A compound of formula I according to claim 4, in which A is phenyl or phenoxyphenyl, $R_a$, $R_b$ and $R_c$ are each independently chlorine or methyl in the 2- and/or 4-position and X is the bridge element

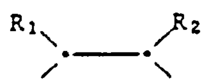

in which $R_1$ is $C_1$-$C_3$alkyl or $CH_2OCH_3$ and $R_2$ is hydrogen.

8. A compound of formula I according to claim I, in which A in combination with $R_a$, $R_b$ and $R_c$ is 2,4-dichlorophenyl, 2-fluoro-4-chlorophenyl, 2-cyano-4-chlorophenyl, 2-methyl-4-chlorophenyl, p-chlorophenoxy, 2-methyl-4-(p-chlorophenoxy) or 2-chloro-4-(p-chlorophenoxy) and $R_1$ to $R_6$ are hydrogen or $C_1$-$C_4$alkyl, but together having not more than 5 C atoms.

9. An isomer or a mixture of isomers of the compounds
5-[2-(2',4'-dichlorophenyl)-4-ethyl-1,3-dioxolan-2-yl]pyrimidine,
5-[2-(2',4'-dichlorophenyl)-4-propyl-1,3-dioxolan-2-yl]pyrimidine,
5-[2-(2',4'-dichlorophenyl)-4-methoxymethyl-1,3-dioxolan-2-yl]pyrimidine,
5-[2-(2',4'-dichlorophenyl)-4,5-dimethyl-1,3-dioxolan-2-yl]pyrimidine,
5-[2-(2',4'-dichlorophenyl)-5,5-dimethyl-1,3-dioxan-2-yl]pyrimidine.

10. An isomer or a mixture of isomers of the compounds
5-[2-(2',4'-dichlorophenyl)-4,6-dimethyl-1,3-dioxan-2-yl]pyrimidine,
5-[2-(2',4'-dichlorophenyl)-4,5,6-trimethyl-1,3-dioxan-2-yl]pyrimidine,
5-[2-(2',4'-dichlorophenyl)-5-ethyl-5-methyl-1,3-dioxan-2-yl]pyrimidine,
5-[2-(2',4'-dichlorophenyl)-4-methyl-1,3-dioxan-2-yl]pyrimidine,
5-[2-(2'-cyano-4'-chlorophenyl)-5-ethyl-5-methyl-1,3-dioxan-2-yl]pyrimidine, 5-[2-(2',4'-dichlorophenyl)-4-ethyl-1,3-dioxan-2-yl]pyrimidine,
5-[2-(2'-chloro-4'-p-chlorophenoxyphenyl)-4-ethyl-1,3-dioxan-2-yl]pyrimidine.

11. A process for the preparation of a compound of formula I according to claim 1, which comprises
A) ketalising a ketone of formula II

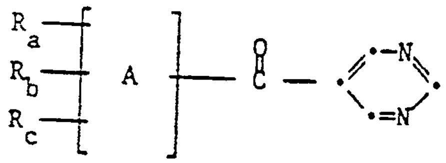

(II)

with a diol of formula III

HO-X-OH (III)

in which A, $R_a$, $R_b$, $R_c$ and X are as defined for formula I, in an inert solvent, or
B) reacting a ketone of formula IV

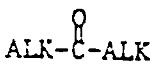

(IV)

with a diol of formula III in an inert solvent to give a compound of formula V

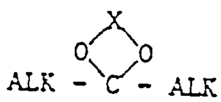

(V)

in which ALK is $C_1$-$C_4$alkyl and X is as defined for formula I, and subsequently ketalising a ketone of formula II using the compound of formula V, which is not isolated from the reaction mixture, in an inert solvent.

**12.** A process according to claim 11, which comprises the addition of an acid in the ketalisation reaction.

**13.** A microbicidal composition for controlling or preventing an attack on plants by microorganisms, which contains as at least one active component a compound of formula I according to claim 1.

**14.** A composition according to claim 13, which contains as active component at least one compound of formula I according to any one of claims 2, 4, 7 or 9.

**15.** A composition according to claim 13, which contains as active component at least one compound of formula I according to any one of claims 3, 5, 6, 8 or 10.

**16.** A method of controlling or preventing an attack on cultivated plants by phytopathogenic microorganisms, which comprises applying to the plant or to the locus thereof or to parts of plants a compound of formula I according to claim 1.

**17.** A method according to claim 16, which comprises applying a compound of formula I according to any one of claims 2, 4, 7 or 9.

**18.** A method according to claim 16, which comprises applying a compound of formula I according to any one of claims 3, 5, 6, 8 or 10.

**19.** A process for the preparation of an agrochemical composition, which comprises intimately mixing at least one compound of formula I according to claim 1 with suitable solid or liquid adjuvants and surfactants.

**Claims for the following Contracting State : AT**

1. A microbicidal composition comprising, in addition to conventional carriers and assistants, at least one compound of the general formula I

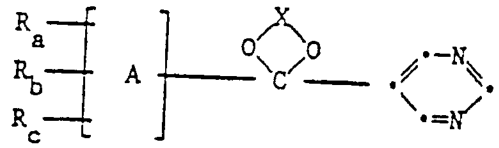

(I)

in which
A is phenyl, phenoxyphenyl, phenylthiophenyl, biphenyl or naphthyl,
$R_a$, $R_b$ and $R_c$ are each independently hydrogen, halogen, cyano, nitro, $C_1$-$C_4$alkyl, $C_1$-$C_4$alkoxy, $C_1$-$C_4$haloalkyl or $C_1$-$C_4$haloalkoxy and
X is one of the following bridge elements

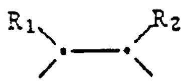

or

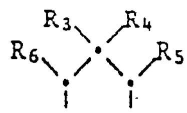

in which $R_1$, and $R_2$ are each independently hydrogen, $C_1$-$C_4$alkyl, $C_1$-$C_4$haloalkyl or $CH_2O$-$C_1$-$C_4$alkyl, $R_4$ is hydrogen, $C_1$-$C_4$alkyl or $CH_2OH$ and $R_3$, $R_5$ and $R_6$ are each independently hydrogen or $C_1$-$C_4$alkyl, or an acid addition salt thereof.

2. A microbicidal composition comprising, in addition to conventional carriers and assistants, at least one compound of formula I in claim 1, in which A, $R_a$, $R_b$, $R_c$, $R_1$ and $R_2$ are as defined above and $R_6$ is hydrogen and $R_3$, $R_4$ and $R_5$ are each independently hydrogen or $C_1$-$C_4$alkyl.

3. A composition according to claim 1, comprising at least one compound of formula I, in which A is phenyl, phenoxyphenyl or phenylthiophenyl, $R_a$, $R_b$ and $R_c$ are each independently hydrogen, halogen, $C_1$-$C_2$alkyl, $C_1$-$C_2$alkoxy, $C_1$-$C_2$haloalkyl, $C_1$-$C_2$haloalkoxy, nitro or cyano, X is one of the bridge elements

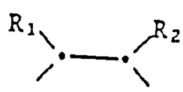

or

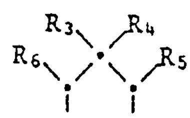

in which $R_1$ and $R_2$ are each independently hydrogen, $C_1$-$C_4$alkyl, $C_1$-$C_2$haloalkyl or $CH_2O$-$C_1$-$C_2$alkyl and $R_4$ is hydrogen, $C_1$-$C_2$alkyl or $CH_2OH$ and $R_3$, $R_5$ and $R_6$ are each independently hydrogen or $C_1$-$C_2$alkyl.

4. A composition according to claim 2, comprising at least one compound of formula I in claim 1, in which A is phenyl, phenoxyphenyl or phenylthiophenyl, $R_a$, $R_b$ and $R_c$ are each independently hydrogen, halogen, $C_1$-$C_2$alkyl, $C_1$-$C_2$alkoxy, $C_1$-$C_2$haloalkyl, $C_1$ -$C_2$haloalkoxy, nitro or cyano, X is one of the bridge elements

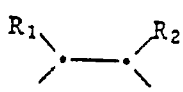

or

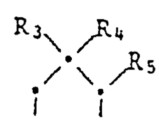

$R_1$ and $R_2$ are each independently hydrogen, $C_1$-$C_4$alkyl, $C_1$-$C_2$haloalkyl or $CH_2$-O-($C_1$-$C_2$)alkyl and $R_3$, $R_4$ and $R_5$ are each independently hydrogen or $C_1$-$C_2$alkyl.

5. A composition according to claim 3, comprising at least one compound of formula I in claim 1, in which A is phenyl or phenoxyphenyl, $R_a$, $R_b$ and $R_c$ are each independently hydrogen, halogen, $C_1$-$C_2$alkyl, $C_1$-$C_2$alkoxy, $C_1$-$C_2$haloalkoxy, nitro or cyano, $R_1$, and $R_2$ are each independently hydrogen, $C_1$-$C_4$alkyl or $CH_2OCH_3$ and $R_4$ is hydrogen, methyl or $CH_2OH$ and $R_3$, $R_5$ and $R_6$ are each independently hydrogen or methyl.

6. A composition according to claim 5, comprising at least one compound of formula I in claim 1, in which A is phenyl or phenoxyphenyl, $R_a$, $R_b$ and $R_c$ are each independently chlorine and/or methyl in the 2- and/or 4-position and X is the bridge element

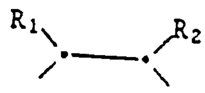

in which $R_1$ is $C_1$-$C_3$alkyl or $CH_2OCH_3$ and $R_2$ is hydrogen.

7. A composition according to claim 4, comprising at least one compound of formula I in claim 1, in which A is phenyl or phenoxyphenyl, $R_a$, $R_b$ and $R_c$ are each independently chlorine or methyl in the 2- and/or 4-position and X is the bridge element

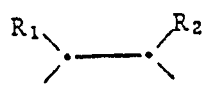

in which $R_1$ is $C_1$-$C_3$alkyl or $CH_2OCH_3$ and $R_2$ is hydrogen.

8. A composition according to claim 6, comprising at least one compound of formula I in claim 1, in which A in combination with $R_a$, $R_b$ and $R_c$ is 2,4-dichlorophenyl, 2-fluoro-4-chlorophenyl, 2-cyano-4-chlorophenyl, 2-methyl-4-chlorophenyl, p-chlorophenoxy, 2-methyl-4-(p-chlorophenoxy) or 2-chloro-4-(p-chlorophenoxy) and $R_1$ to $R_6$ are hydrogen or $C_1$-$C_4$alkyl, but together having not more than 5 C atoms.

9. A composition according to claim 2, comprising at least one isomer or a mixture of isomers of the compounds
5-[2-(2',4'-dichlorophenyl)-4-ethyl-1,3-dioxolan-2-yl]pyrimidine,
5-[2-(2',4'-dichlorophenyl)-4-propyl-1,3-dioxolan-2-yl]pyrimidine,
5-[2-(2',4'-dichlorophenyl)-4-methoxymethyl-1,3-dioxolan-2-yl]pyrimidine,
5-[2-(2',4'-dichlorophenyl)-4,5-dimethyl-1,3-dioxolan-2-yl]pyrimidine,
5-[2-(2',4'-dichlorophenyl)-5,5-dimethyl-1,3-dioxan-2-yl]pyrimidine.

10. A composition according to claim 1, comprising at least one isomer or a mixture of isomers of the compounds
5-[2-(2',4'-dichlorophenyl)-4,6-dimethyl-1,3-dioxan-2-yl]pyrimidine,
5-[2-(2',4'-dichlorophenyl)-4,5,6-trimethyl-1,3-dioxan-2yl]pyrimidine,
5-[2-(2'-4'-dichlorophenyl)-5-ethyl-5-methyl-1,3-dioxan-2-yl]pyrimidine,
5-[2-(2',4'-dichlorophenyl)-4-methyl-1,3-dioxan-2-yl]pyrimidine,
5-[2-(2'-cyano-4'-chlorophenyl)-5-ethyl-5-methyl-1,3-dioxan-2-yl]pyrimidine,
5-[2-(2',4'-dichlorophenyl)-4-ethyl-1,3-dioxan-2-yl]pyrimidine,
5-[2-(2'-chloro-4'-p-chlorophenoxyphenyl)-4-ethyl-1,3-dioxan-2-yl]pyrimidine.

11. A process for the preparation of a compound of formula I in claim 1, which comprises
A) ketalising a ketone of formula II

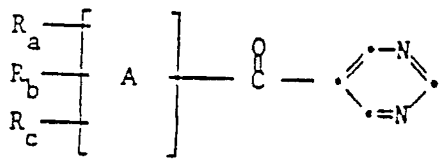

(II)

with a diol of formula III

HO-X-OH (III)

in which A, $R_a$, $R_b$,$R_c$ and X are as defined for formula I, in an inert solvent, or
B) reacting a ketone of formula IV

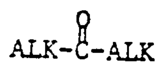

(IV)

with a diol of formula III, in an inert solvent, to give a compound of formula V

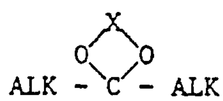

(V)

in which ALK is $C_1$-$C_4$alkyl and X is as defined for formula I, and subsequently ketalising a ketone of formula II using the compound of formula V, which is not isolated from the reaction mixture, in an inert solvent.

**12.** A process according to claim 11, which comprises the addition of an acid in the ketalisation reaction.

**13.** A composition according to any one of claims 1-10 for controlling or preventing an attack on plants by microorganisms.

**14.** A method of controlling or preventing an attack on cultivated plants by phytopathogenic microorganisms, which comprises applying to the plant or to the locus thereof or to parts of plants a compound of formula I defined according to claim 1.

**15.** A method according to claim 14, which comprises applying a compound of formula I defined according to any one of claims 2, 4, 7 or 9.

**16.** A method according to claim 14, which comprises applying a compound of formula I defined according to any one of claims 3, 5, 6, 8 or 10.

**17.** The use of a compound of formula I prepared according to claim 11 for controlling and/or for preventive treatment of an attack on plants by microorganisms.

**18.** The use according to claim 17 of a compound of formula I defined in any one of claims 2, 4, 7 or 9.

**19.** The use according to claim 17 of a compound of formula I defined in any one of claims 3, 5, 6, 8 or 10.

**20.** The use according to any one of claims 17 to 19, in which the microorganisms are phytopathogenic fungi.

**Revendications**

1. Composé de formule générale I

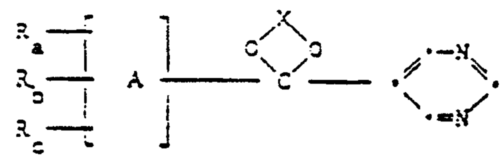

(I)

dans laquelle
A représente un phényle, un phénoxyphényle, un phénylthiophényle, un biphényle ou un naphtyle,
$R_a$, $R_b$ et $R_c$, indépendamment les uns des autres, représentent chacun un hydrogène, un halogène, un cyano, un nitro, un alkyle en $C_1$-$C_4$, un alcoxy en $C_1$-$C_4$, un haloalkyle en $C_1$-$C_4$ ou un haloalcoxy en $C_1$-$C_4$, et
X représente un des éléments de pontage suivants

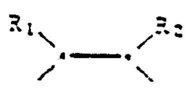

ou

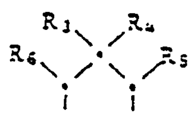

où
$R_1$ et $R_2$, indépendamment l'un de l'autre, représentent chacun un hydrogène, un alkyle en $C_1$-$C_4$, un haloalkyle en $C_1$-$C_4$ ou un $CH_2O$-alkyle en $C_1$-$C_4$ et
$R_4$ représente un hydrogène, un alkyle en $C_1$-$C_4$ ou $CH_2OH$ et
$R_3$, $R_5$ et $R_6$, indépendamment les uns des autres, représentent un hydrogène ou un alkyle en $C_1$-$C_4$, y compris leurs sels d'additions acides.

2. Composé de formule I selon la revendication 1 dans lequel
A, $R_a$, $R_b$, $R_c$, $R_1$ et $R_2$ ont la signification donnée, tandis que $R_6$ est un hydrogéne et $R_3$, $R_4$ et $R_5$, indépendamment les uns des autres représentent un hydrogène ou un alkyle en $C_1$-$C_4$.

3. Composé de formule I selon la revendication 1, dans lequel
A représente un phényle, un phénoxyphényle ou un phénylthiophényle, $R_a$, $R_b$ et $R_c$, indépendamment les uns des autres, représentent un hydrogène, un halogène, un alkyle en $C_1$-$C_2$, un alcoxy en $C_1$-$C_2$, un haloalkyle en $C_1$-$C_2$, un haloalcoxy en $C_1$-$C_2$, un nitro ou un cyano, X représente un des éléments de pontage

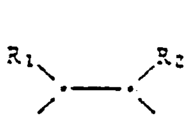

ou

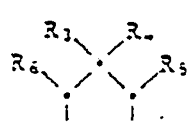

où $R_1$ et $R_2$, indépendamment l'un de l'autre, représentent un hydrogène, un alkyle en $C_1$-$C_4$, un haloalkyle en $C_1$-$C_2$ ou un $CH_2O$-alkyle en $C_1$-$C_2$, et $R_4$ représente un hydrogène, un alkyle en $C_1$-$C_2$ ou $CH_2OH$ et $R_3$, $R_5$ et $R_6$, indépendamment les uns des autres, représentent un hydrogène ou un alkyle en $C_1$-$C_2$.

4. Composé de formule I selon la revendication 2, dans lequel
A représente un phényle, un phénoxyphényle ou un phénylthiophényle, $R_a$, $R_b$ et $R_c$, indépendamment les uns des autres, représentent un hydrogène, un halogène, un alkyle en $C_1$-$C_2$, un alcoxy en $C_1$-$C_2$, un haloalkyle en $C_1$-$C_2$, un haloalcoxy en $C_1$-$C_2$, un nitro ou un cyano, X représente un des éléments de pontage

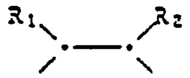 ou 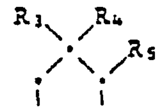

où $R_1$ et $R_2$, indépendamment l'un de l'autre, représentent un hydrogène, un alkyle en $C_1$-$C_4$, un haloalkyle en $C_1$-$C_2$ ou un $CH_2O$-alkyle en $C_1$-$C_2$, et $R_3$, $R_4$ et $R_5$, indépendamment les uns des autre, représentent un hydrogène ou un alkyle en $C_1$-$C_2$.

**5.** Composé de formule I selon la revendication 3, dans lequel A représente un phényle ou un phénoxyphényle, $R_a$, $R_b$ et $R_c$, indépendamment les uns des autres, représentent un hydrogène, un halogène, un alkyle en $C_1$-$C_2$, un alcoxy en $C_1$-$C_2$, un haloalcoxy en $C_1$-$C_2$, un nitro ou un cyano, $R_1$ et $R_2$, indépendamment l'un de l'autre, représentent un hydrogène, un alkyle en $C_1$-$C_4$ ou $CH_2OCH_3$ et $R_4$ représente un hydrogène, un méthyle ou $CH_2OH$ et $R_3$, $R_5$ et $R_6$, indépendamment les uns des autres, représentent un hydrogène ou un méthyle.

**6.** Composé de formule I selon la revendication 5, dans lequel A représente un phényle ou un phénoxyphényle, $R_a$, $R_b$ et $R_c$, indépendamment les uns des autres, représentent un Cl et/ou un méthyle, en occupant la position 2 et/ou 4, et X représente l'élément de pontage où $R_1$ représente un alkyle en $C_1$-$C_3$

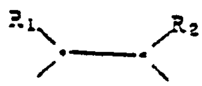

ou $CH_2OCH_3$ et $R_2$ représente un hydrogène.

**7.** Composé de formule I selon la revendication 4, dans lequel A représente un phényle ou un phénoxyphényle; $R_a$ , $R_b$ et $R_c$, indépendamment les uns des autres, représentent un chlore ou un méthyle en occupant la position 2 et/ou 4, et X représente l'élément de pontage

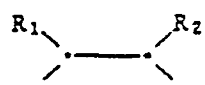

où $R_1$ représente un alkyle en $C_1$-$C_3$ ou $CH_2OCH_3$ et $R_2$ représente un hydrogène.

**8.** Composé de formule I selon la revendication 1, dans lequel A en combinaison avec $R_a$, $R_b$ et $R_c$ représente un 2,4-dichlorophényle, un 2-fluoro-4-chlorophényle, un 2-cyano-4-chlorophényle, un 2-méthyl-4-chlorophényle, un p-chlorophénoxy, un 2-méthyl-4-(p-chlorophénoxy) ou un 2-chloro-4-(p-chlorophénoxy),tandis que $R_1$ à $R_6$ représentent un hydrogène ou un alkyle en $C_1$-$C_4$, mais en ayant ensemble au plus 5 atomes de C.

**9.** Un isomère ou un mélange des isomères des composés
5-[2-(2',4'-dichlorophényl)-4-éthyl-1,3-dioxolanne-2-yl]-pyrimidine,
5-[2-(2',4'-dichlorophényl)-4-propyl-1,3-dioxolanne-2-yl]-pyrimidine,
5-[2-(2',4'-dichlorophényl)-4-méthoxyméthyl-1,3-dioxolanne-2-yl]-pyrimidine,
5-[2-(2',4'-dichlorophényl)-4,5-diméthyl-1,3-dioxolanne-2-yl]-pyrimidine,
5-[2-(2',4'-dichlorophényl)-5,5-diméthyl-1,3-dioxanne-2-yl]-pyrimidine.

**10.** Un isomère ou un mélange des isomères des composés
5-[2-(2',4'-dichlorophényl)-4,6-diméthyl-1,3-dioxanne-2-yl]-pyrimidine,
5-[2-(2',4'-dichlorophényl)-4,5,6-triméthyl-1,3-dioxanne-2-yl]-pyrimidine,
5-[2-(2',4'-dichlorophényl)-5-éthyl-5-méthyl-1,3-dioxanne-2-yl]-pyrimidine,
5-[2-(2',4'-dichlorophényl)-4-méthyl-1,3-dioxanne-2-yl]-pyrimidine,
5-[2-(2'-cyano-4'-chlorophényl)-5-éthyl-5-méthyl-1,3-dioxanne -2-yl]-pyrimidine,

5-[2-(2',4'-dichlorophényl)-4-éthyl-1,3-dioxanne-2-yl]-pyrimidine,
5-[2-(2'-chloro-4'-p-chlorophénoxyphényl)-4-éthyl-1,3-dioxanne-2-yl]-pyrimidine.

**11.** Procédé pour préparer le composé de formule I selon la revendication 1, caractérisé

A) par la réaction d'acétalisation d'une cétone de formule II

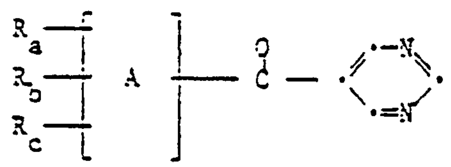

(II)

avec un diol de formule III

HO-X-OH (III)

où A, $R_a$, $R_b$, $R_c$ et X ont les significations données pour la formule I, dans un solvant inerte, ou

B) par la réaction d'une cétone de formule IV

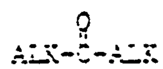

(IV)

avec un diol de formule III dans un solvant inerte pour obtenir un composé de formule V

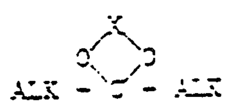

(V)

où ALK représente un alkyle en $C_1$-$C_4$ et X a la signification donnée pour la formule I, suivie d'une acétalisation d'une cétone de formule II au moyen du composé de formule V, non isolé à partir du mélange réactionnel, dans un solvant inerte.

**12.** Procédé selon la revendication 11, caractérisé par l'addition d'un acide pour la réaction d'acétalisation.

**13.** Agent microbicide pour lutter contre, ou pour prévenir, une attaque de plantes par des micro-organismes, caractérisé en ce qu'il comporte au moins un composant actif, sous forme d'un composé de formule I conforme à la revendication 1.

**14.** Agent selon la revendication 13, caractérisé en ce qu'il comporte, comme composant actif, au moins un composé de formule I conforme à l'une des revendications 2, 4, 7 ou 9.

**15.** Agent selon la revendication 13, caractérisé en ce qu'il comporte, comme composant actif, au moins un composé de formule I conforme à l'une des revendications 3, 5, 6, 8 ou 10.

**16.** Procédé pour lutter contre, ou pour prévenir, une attaque de plantes de culture par des micro-organismes phytopathogènes, caractérisé en ce que l'on applique un composé de formule I, conforme à la revendication 1, sur la plante ou sur son emplacement ou sur des parties de la plante.

**17.** Procédé selon la revendication 16, caractérisé en ce que l'on applique un composé de formule I conforme à l'une des revendications 2, 4, 7 ou 9.

**18.** Procédé selon la revendication 16, caractérisé en ce que l'on applique un composé de formule 1 conforme à l'une des revendications 3, 5, 6, 8 ou 10.

**19.** Procédé pour préparer un agent agrochimique, caractérisé en ce que l'on mélange de façon intime au moins un composé de formule I conforme à la revendication 1 avec des additifs et des agents tensio-actifs appropriés, solides ou liquides.

**Revendications pour l'Etat contractant suivant : AT**

**1.** Agent microbicide comportant, en plus des additifs et supports habituels, au moins un composé de formule générale I

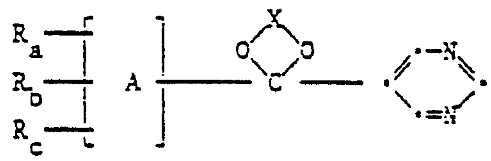 (I) ,

dans laquelle
A représente un phényle, un phénoxyphényle, un phénylthiophényle, un biphényle ou un naphtyle,
$R_a$, $R_b$ et $R_c$, indépendamment les uns des autres, représentent chacun un hydrogène, un halogène, un cyano, un nitro, un alkyle en $C_1$-$C_4$, un alcoxy en $C_1$-$C_4$, un haloalkyle en $C_1$-$C_4$ ou un haloalcoxy en $C_1$-$C_4$, et
X représente un des éléments de pontage suivants

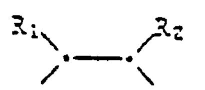 ou 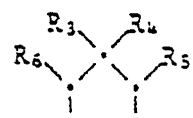

où
$R_1$ et $R_2$, indépendamment l'un de l'autre, représentent chacun un hydrogène, un alkyle en $C_1$-$C_4$, un haloalkyle en $C_1$-$C_4$ ou un $CH_2O$-alkyle en $C_1$-$C_4$ et
$R_4$ représente un hydrogène, un alkyle en $C_1$-$C_4$ ou $CH_2OH$ et
$R_3$, $R_5$ et $R_6$, indépendamment les uns des autres, représentent un hydrogène ou un alkyle en $C_1$-$C_4$, y compris leurs sels d'additions acides.

**2.** Agent microbicide comportant, en plus des additifs et supports habituels, au moins un composé de formule I dans la revendication 1, où
A, $R_a$, $R_b$, $R_c$, $R_1$ et $R_2$ ont la signification donnée, tandis que $R_6$ est un hydrogène et $R_3$, $R_4$ et $R_5$, indépendamment les uns des autres représentent un hydrogène ou un alkyle en $C_1$-$C_4$.

**3.** Agent selon la revendication 1, comportant au moins un composé de formule I, dans lequel
A représente un phényle, un phénoxyphényle ou un phénylthiophényle, $R_a$, $R_b$ et $R_c$, indépendamment les uns des autres, représentent un hydrogène, un halogène, un alkyle en $C_1$-$C_2$, un alcoxy en $C_1$-$C_2$, un haloalkyle en $C_1$-$C_2$, un haloalcoxy en $C_1$-$C_2$, un nitro ou un cyano. X représente un des éléments de pontage

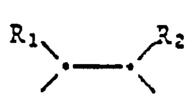 ou 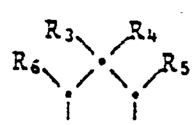

où $R_1$ et $R_2$, indépendamment l'un de l'autre, représentent un hydrogène, un alkyle en $C_1$-$C_4$, un haloalkyle on $C_1$-$C_2$ ou un $CH_2O$-alkyle en $C_1$-$C_2$, et $R_4$ représente un hydrogène, un alkyle en $C_1$-$C_2$ ou $CH_2OH$ et $R_3$, $R_5$ et $R_6$, indépendamment les uns des autres, représentent un hydrogène ou un alkyle en $C_1$-$C_2$.

4. Agent selon la revendication 2, comportant au moins un composé de formule I dans la revendication 1, dans lequel A représente un phényle, un phénoxyphényle ou un phénylthiophényle, $R_a$, $R_b$ et $R_c$, indépendamment les uns des autres, représentent un hydrogène, un halogène, un alkyle en $C_1$-$C_2$, un alcoxy en $C_1$-$C_2$, un haloalkyle en $C_1$-$C_2$, un haloalcoxy en $C_1$-$C_2$, un nitro ou un cyano, X représente un des éléments de pontage

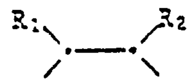 ou 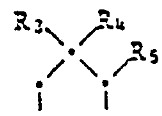

où $R_1$ et $R_2$, indépendamment l'un de l'autre, représentent un hydrogène, un alkyle en $C_1$-$C_4$, un haloalkyle en $C_1$-$C_2$ ou un $CH_2O$-alkyle en $C_1$-$C_2$, et $R_3$, $R_4$ et $R_5$, indépendamment les uns des autre, représentent un hydrogène ou un alkyle en $C_1$-$C_2$.

5. Agent selon la revendication 3, comportant au moins un composé de formule I dans la revendication 1, dans lequel A représente un phényle ou un phénoxyphényle, $R_a$, $R_b$ et $R_c$, indépendamment les uns des autres, représentent un hydrogène, un halogène, un alkyle en $C_1$-$C_2$, un alcoxy en $C_1$-$C_2$, un haloalcoxy en $C_1$-$C_2$, un nitro ou un cyano, $R_1$ et $R_2$, indépendamment l'un de l'autre, représentent un hydrogène, un alkyle en $C_1$-$C_4$ ou $CH_2OCH_3$ et $R_4$ représente un hydrogène, un méthyle ou $CH_2OH$ et $R_3$, $R_5$ et $R_6$, indépendamment les uns des autres, représentent un hydrogène ou un méthyle.

6. Agent selon la revendication 5, comportant au moins un composé de formule I dans la revendication 1, dans lequel A représente un phényle ou un phénoxyphényle, $R_a$, $R_b$ et $R_c$, indépendamment les uns des autres, représentent un Cl et/ou un méthyle, en occupant la position 2 et/ou 4, et X représente l'élément de pontage

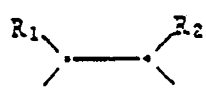

où $R_1$ représente un alkyle en $C_1$-$C_3$ ou $CH_2OCH_3$ et $R_2$ représente un hydrogène.

7. Agent selon la revendication 4, comportant au moins un composé de formule I dans la revendication 1, dans lequel A représente un phényle ou un phénoxyphényle; $R_a$, $R_b$ et $R_c$, indépendamment les uns des autres, représentent un chlore ou un méthyle en occupant la position 2 et/ou 4, et X représente l'élément de pontage

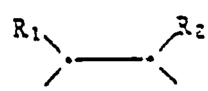

où $R_1$ représente un alkyle en $C_1$-$C_3$ ou $CH_2OCH_3$ et $R_2$ représente un hydrogène.

8. Agent selon la revendication 6, comportant au moins un agent comportant au moins un composé de formule I dans la revendication 1, dans lequel A en combinaison avec $R_a$, $R_b$ et $R_c$ représente un 2,4-dichlorophényle, un 2-fluoro-4-chlorophényle, un 2-cyano-4-chlorophényle, un 2-méthyl-4-chlorophényle, un p-chlorophénoxy, un 2-méthyl-4-(p-chlorophénoxy) ou un 2-chloro-4-(p-chlorophénoxy),tandis que $R_1$ à $R_6$ représentent un hydrogène ou un alkyle en $C_1$-$C_4$, mais en ayant ensemble au plus 5 atomes de C.

9. Agent selon la revendication 2, comportant au moins un isomère ou un mélange d'isomères des composés
5-[2-(2',4'-dichlorophényl)-4-éthyl-1,3-dioxolanne-2-yl]-pyrimidine,
5-[2-(2',4'-dichlorophényl)-4-propyl-1,3-dioxolanne-2-yl]-pyrimidine,

5-[2-(2',4'-dichlorophényl)-4-méthoxyméthyl-1,3-dioxolanne-2-yl]-pyrimidine,
5-[2-(2',4'-dichlorophényl)-4,5-diméthyl-1,3-dioxolan-ne-2-yl]-pyrimidine,
5-[2-(2',4'-dichlorophényl)-5,5-diméthyl-1,3-dioxanne-2-yl]-pyrimidine.

**10.** Agent selon la revendication 1, comportant au moins un isomère ou un mélange d'isomères des composés
5-[2-(2',4'-dichlorophényl)-4,6-diméthyl-1,3-dioxanne-2yl]-pyrimidine,
5-[2-(2',4'-dichlorophényl)-4,5,6-triméthyl-1,3-dioxanne-2-yl]-pyrimidine,
5-[2-(2',4'-dichlorophényl)-5-éthyl-5-méthyl-1,3-dioxanne-2-yl]-pyrimidine,
5-[2-(2',4'-dichlorophényl)-4-méthyl-1,3-dioxanne-2-yl]-pyrimidine,
5-[2-(2'-cyano-4'-chlorophényl)-5-éthyl-5-méthyl-1,3-dioxanne -2-yl]-pyrimidine,
5-[2-(2',4'-dichlorophényl)-4-éthyl-1,3-dioxanne-2-yl]-pyrimidine,
5-[2-(2'-chloro-4'-p-chlorophénoxyphényl)-4-éthyl-1,3-dioxanne-2-yl]-pyrimidine.

**11.** Procédé pour préparer le composé de formule I selon la revendication 1, caractérisé
A) par la réaction d'acétalisation d'une cétone de formule II

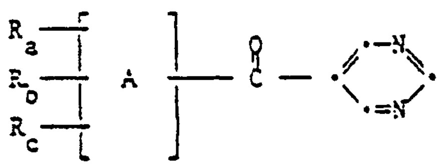

(II)

avec un diol de formule III

HO-X-OH (III)

où A, $R_a$, $R_b$, $R_c$ et X ont les significations données pour la formule I, dans un solvant inerte, ou
B) par la réaction d'une cétone de formule IV

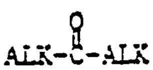

(IV)

avec un diol de formule III dans un solvant inerte pour obtenir un composé de formule V

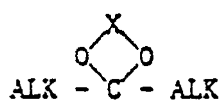

(V)

où ALK représente un alkyle en $C_1$-$C_4$ et X a la signification donnée pour la formule I, suivie d'une acétalisation d'une cétone de formule II au moyen du composé de formule V, non isolé à partir du mélange réactionnel, dans un solvant inerte.

**12.** procédé selon la revendication 11, caractérisé par l'addition d'acide pour la réaction d'acétalisation.

**13.** Agent selon l'une des revendications 1 à 10 pour lutter contre, ou pour prévenir, une attaque de plantes par des micro-organismes.

**14.** Procédé pour lutter contre, ou pour prévenir, une attaque de plantes de culture par des micro-organismes phytopathogènes, caractérisé en ce que l'on applique un composé de formule I ,défini selon la revendication 1, sur la plante ou sur son emplacement ou sur des parties de la plante.

**15.** Procédé selon la revendication 14, caractérisé en ce que l'on applique un composé de formule I défini

conformément à l'une des revendications 2, 4, 7 ou 9.

**16.** Procédé selon la revendication 14, caractérisé en ce que l'on applique un composé de formule I défini conformément à l'une des revendications 3, 5, 6, 8 ou 10.

**17.** Utilisation de composés préparés conformément à la revendication 11 et de formule I pour lutter contre et/ou pour prévenir une attaque de plantes par des micro-organismes.

**18.** Utilisation selon la revendication 17 d'un composé de formule I défini dans une des revendications 2, 4, 7 ou 9.

**19.** Utilisation selon la revendication 17 de composés de formule I définis dans une des revendications 3, 5, 6, 8 ou 10.

**20.** Utilisation selon l'une des revendications 17 à 19, caractérisée en ce qu'il s'agit, pour les micro-organismes, de champignons phytopathogènes.